# EUROPEAN PATENT SPECIFICATION

(11) **EP 0 555 438 B1**
(45) Date of publication and mention of the grant of the patent: **22.01.1997**
(21) Application number: 92917106.4
(22) Date of filing: 03.08.1992
(51) Int. Cl.: C12N 15/31, C12N 5/10, C12N 1/21, C07K 14/00

(54) **STREPTOLYSIN O VARIANTS**
STREPTOLYSIN O VARIANTE
VARIANTS DE STREPTOLYSINE O

(30) Priority: 30.08.1991 US 752428
(43) Date of publication of application: 18.08.1993
(73) Proprietor: BECKMAN INSTRUMENTS, INC., Fullerton, CA 92634 (US)
(72) Inventor: ADAMS, Craig, W., Corona, CA 91720 (US)
(74) Representative: Ede, Eric
(86) International application number: PCT/US92/06380
(87) International publication number: WO 93/05155

(56) References cited:
- EP-A- 0 369 825
- GB-A- 2 233 977
- INFECTION & IMMUNITY, vol. 55, no. 12, December 1987, Washington, DC (US); M. KEHOE et al., pp. 3228-3232

### SEQUENCE LISTING

### (1) GENERAL INFORMATION:

(i) APPLICANT: Adams, Craig M.
(ii) TITLE OF INVENTION: Streptolysin O Variants
(iii) NUMBER OF SEQUENCES: 2
(iv) CORRESPONDENCE ADDRESS:
   (A) ADDRESSEE: Beckman Instruments, Inc.
   (B) STREET: 2500 Harbor Blvd.
   (C) CITY: Fullerton
   (D) STATE: California
   (E) COUNTRY: USA
   (F) ZIP: 92634
(v) COMPUTER READABLE FORM:
   (A) MEDIUM TYPE: Diskette, 3.5 inch, 1.44 Mb
   (B) COMPUTER: IBM
   (C) Operating System: MS.DOS
   (D) SOFTWARE: WordPerfect 5.1
(vi) CURRENT APPLICATION DATA:
   (A) APPLICATION NUMBER: 07/752,428
   (B) FILING DATE: August 30, 1991
   (C) CLASSIFICATION:
(vii) PRIOR APPLICATION DATA:
   (A) APPLICATION NUMBER:
   (B) REGISTRATION NUMBER:
   (C) FILING DATE:
(viii) ATTORNEY/AGENT INFORMATION:
   (A) NAME: Burgoon, Richard P.
   (B) REGISTRATION NUMBER: 34,787
   (C) REFERENCE/DOCKET NUMBER: 128D-1028
(ix) TELECOMMUNICATION INFORMATION:
   (A) TELEPHONE: (714) 773-7610
   (B) TELEFAX: (714) 773-7936

### (2) INFORMATION FOR SEQ ID NO.: 1:

(i) SEQUENCE CHARACTERISTICS:
   (A) LENGTH: 1524 base pairs
   (B) TYPE: Nucleic acid
   (C) STRANDEDNESS: single
   (D) TOPOLOGY: linear
(ii) MOLECULAR TYPE: genomic DNA
(vi) ORIGINAL SOURCE:
   (A) ORGANISM: *Streptococcus pyogenes*
(vii) IMMEDIATE SOURCE:
   (A) LIBRARY: genomic
   (B) CLONE: mSLO.3/6
(xi) SEQUENCE DESCRIPTION: SEQ ID NO.: 1:
   ATG GAT CCG TCA GAA GAC AAA AAA AAG AGC GAA GAA GAT CAC ACT GAA 48
   GAA ATC AAT GAC AAG ATT TAT TCA CTA AAT TAT AAT GAG CTT GAA GTA 96
   CTT GCT AAA AAT GGT GAA ACC ATT GAA AAT TTT GTT CCT AAA GAA GGC 144
   GTT AAG AAA GCT GAT AAA TTT ATT GTC ATT GAA AGA AAG AAA AAA AAT 192
   ATC AAC ACT ACA CCA GTC GAT ATT TCC ATC ATT GAC TCT GTC ACT GAT 240
   AGG ACC TAT CCA GCA GCC CTT CAG CTG GCT AAT AAA GGT TTT ACC GAA 288
   AAC AAA CCA GAC GCG GTA GTC ACC AAG CGA AAC CCA CAA AAA ATC CAT 336
   ATT GAT TTA CCA GGT ATG GGA GAC AAA GCA ACG GTT GAG GTC AAT GAC 384
   CCT ACC TAT GCC AAT GTT TCA ACA GCT ATT GAT AAT CTT GTT AAC CAA 432
   TGG CAT GAT AAT TAT TCT GGT GGT AAT ACG CTT CCT GCC AGA ACA CAA 480
   TAT ACT AAA TCA ATG GTA TAT TCT AAG TCA CAG ATT GAA GCA GCT CTA 528
   AAT GTT AAT AGC AAA ATC TTA GAT GGT ACT TTA GGC ATT GAT TTC AAG 576
   TCG ATT TCA AAA GGT GAA AAG AAG GTG ATG ATT GCA GCA TAC AAG CAA 624
   ATT TTT TAC ACC GTA TCA GCA AAC CTT CCT AAT AAT CCT GCG GAT GTG 672
   TTT GAT AAA TCA GTG ACC TTT AAA GAG TTG CAA CGA AAA GGT GTC AGC 720
   AAT GAA GCT CCG CCA CTC TTT GTG AGT AAC GTA GCC TAT GGT CGA ACT 768
   GTT TTT GTC AAA CTA GAA ACA AGT TCT AAA AGT AAT GAT GTT GAA GCG 816
   GCC TTT AGT GCA GCT CTA AAA GGA ACA GAT GTT AAA ACT AAT GGA AAA 864
   TAC TCT GAT ATC TTA GAA AAT AGC TCA TTT ACA GCT GTC GTT TTA GGA    912
   GGA GAT GCT GCA GAG CAC AAT AAG GTA GTC ACA AAA GAC TTT GAT GTT    960
   ATT AGA AAC GTT ATC AAA GAC AAT GCT ACC TTC AGT AGA AAA AAC CCA   1008
   GCT TAT CCT ATT TCA TAC ACC AGT GTT TTC CTT AAA AAT AAT AAA ATT   1056
   GCG GGT GTC AAT AAC AGA ACT GAA TAC GTT GAA ACA ACA TCT ACC GAG   1104
   TAC ACT AGT GGA AAA ATT AAC CTG TCT CAT CAA GGC GCG TAT GTT GCT   1152
   CAA TAT GAA ATC CTT TGG GAT GAA ATC AAT TAT GAT GAC AAA GGA AAA   1200
   GAA GTG ATT ACA AAA CGA CGT TGG GAT AAC AAC TGG TAT AGT AAG ACA   1248
   TCA CCA TTT AGC ACA GTT ATC CCA CTA GGA GCT AAT TCA CGA AAT ATA   1296
   CGT ATC ATG GCT AGA GAG TGC ACC GGC TTA GCT TGG GAA TGG TGG CGA   1344
   AAA GTG ATC GAC GAA AGA GAT GTG AAA CTG TCT AAA GAA ATC AAT GTC   1392
   AAC ATC TCA GGA TCA ACC CTG AGC CCA TAT GGT TCG ATT ACT TAT AAG   1440
   TAG GAC TGG TTC AAG AGG TTC GTC AAG CAC CTT GAT GCT GCT TAT CTC   1488
   TTG AGA TCC CCG GGT AGG CCT AGT TAA CTA GTC GAC                   1524

### (3) INFORMATION FOR SEQ ID NO.: 2:

(i) SEQUENCE CHARACTERISTICS:
   (A) LENGTH: 480 amino acids
   (B) TYPE: Amino Acid
   (D) TOPOLOGY: linear
(ii) MOLECULAR TYPE: protein
(ix) FEATURE:
   (A) NAME/KEY: Signal sequence
   (B) LOCATION: amino acid 98 to amino acid 571 of SLO (except for mutation at amino acid 304 of following sequence)
   (C) IDENTIFICATION METHOD: experimentally determined based upon production of soluble, non-hemolytically active SLO from recombinant vector
   (D) OTHER INFORMATION: Does not lyse red blood cells
(xi) SEQUENCE DESCRIPTION: SEQ ID NO.: 2:
   Met Asp Pro Ser Glu Asp Lys Lys Lys Ser Glu Glu Asp His Thr Glu
   -2 -1 5 10
   Glu Ile Asn Asp Lys Ile Tyr Ser Leu Asn Tyr Asn Glu Leu Glu Val
   15 20 25 30
   Leu Ala Lys Asn Gly Glu Thr Ile Glu Asn Phe Val Pro Lys Glu Gly
   35 40 45
   Val Lys Lys Ala Asp Lys Phe Ile Val Ile Glu Arg Lys Lys Lys Asn
   50 55 60
   Ile Asn Thr Thr Pro Val Asp Ile Ser Ile Ile Asp Ser Val Thr Asp
   65 70 75
   Arg Thr Tyr Pro Ala Ala Leu Gln Leu Ala Asn Lys Gly Phe Thr Glu
   80 85 90
   Asn Lys Pro Asp Ala Val Val Thr Lys Arg Asn Pro Gln Lys Ile His
   95 100 105 110
   Ile Asp Leu Pro Gly Met Gly Asp Lys Ala Thr Val Glu Val Asn Asp
   115 120 125
   Pro Thr Tyr Ala Asn Val Ser Thr Ala Ile Asp Asn Leu Val Asn Gln
   130 135 140
   Trp His Asp Asn Tyr Ser Gly Gly Asn Thr Leu Pro Ala Arg Thr Gln
   145 150 155
   Tyr Thr Lys Ser Met Val Tyr Ser Lys Ser Gln Ile Glu Ala Ala Leu
   160 165 170
   Asn Val Asn Ser Lys Ile Leu Asp Gly Thr Leu Gly Ile Asp Phe Lys
   175 180 185 190
   Ser Ile Ser Lys Gly Glu Lys Lys Val Met Ile Ala Ala Tyr Lys Gln
   195 200 205
   Ile Phe Tyr Thr Val Ser Ala Asn Leu Pro Asn Asn Pro Ala Asp Val
   210 215 220
   Phe Asp Lys Ser Val Thr Phe Lys Glu Leu Gln Arg Lys Gly Val Ser
   225 230 235
   Asn Glu Ala Pro Pro Leu Phe Val Ser Asn Val Ala Tyr Gly Arg Thr
   240 245 250
   Val Phe Val Lys Leu Glu Thr Ser Ser Lys Ser Asn Asp Val Glu Ala
   255 260 265 270
   Ala Phe Ser Ala Ala Leu Lys Gly Thr Asp Val Lys Thr Asn Gly Lys
   275 280 285
   Tyr Ser Asp Ile Leu Glu Asn Ser Ser Phe Thr Ala Val Val Leu Gly
   290 295 300
   Gly Asp Ala Ala Glu His Asn Lys Val Val Thr Lys Asp Phe Asp Val
           305                 310                 315
   Ile Arg Asn Val Ile Lys Asp Asn Ala Thr Phe Ser Arg Lys Asn Pro
       320                 325                 330
   Ala Tyr Pro Ile Ser Tyr Thr Ser Val Phe Leu Lys Asn Asn Lys Ile
   335                 340                 345                 350
   Ala Gly Val Asn Asn Arg Thr Glu Tyr Val Glu Thr Thr Ser Thr Glu
                   355                 360                 365
   Tyr Thr Ser Gly Lys Ile Asn Leu Ser His Gln Gly Ala Tyr Val Ala
               370                 375                 380
   Gln Tyr Glu Ile Leu Trp Asp Glu Ile Asn Tyr Asp Asp Lys Gly Lys
           385                 390                 395
   Glu Val Ile Thr Lys Arg Arg Trp Asp Asn Asn Trp Tyr Ser Lys Thr
       400                 405                 410
   Ser Pro Phe Ser Thr Val Ile Pro Leu Gly Ala Asn Ser Arg Asn Ile
   415                 420                 425                 430
   Arg Ile Met Ala Arg Glu Cys Thr Gly Leu Ala Trp Glu Trp Trp Arg
                   435                 440                 445
   Lys Val Ile Asp Glu Arg Asp Val Lys Leu Ser Lys Glu Ile Asn Val
               450                 455                 460
   Asn Ile Ser Gly Ser Thr Leu Ser Pro Tyr Gly Ser Ile Thr Tyr Lys
           465                 470                 475

## Description

### RELATED APPLICATIONS

This application is related to the invention disclosed in WO93/05156 entitled "Streptolysin O Derivatives" by Craig W Adams and Eva Y Wang and that of WO93/05152 entitled "Antibodies to Streptolysin O Derivatives and variants".

### FIELD OF THE INVENTION

The present invention is generally related to Streptolysin O and more particularly to Streptolysin O variants produced by recombinant DNA technology.

### BACKGROUND OF THE INVENTION

Disclosed herein is a variant version of the antigenic substance, Streptolysin O. Streptolysin O is associated in humans with, for example, rheumatic fever, such that immunodiagnostic assays for evidence of immunological response against Streptolysin O are routinely utilized. The disclosed variant version of Streptolysin O is produced by recombinant DNA techniques, is soluble upon expression, and has substantially no hemolytic activity. Prior to this invention, Streptolysin O could be obtained via the bacteria *Streptococcus pyogenes.* However, Streptolysin O, in its wild-type form, is a dangerous substance in that it will lyse cells, lyse neutrophils, inhibit lymphocyte transformation, and release histimine from plateletes. The toxic and pathogenic properties of Streptolysin O are typically monitored by the lysis of red blood cells.

### I. The Genetic Code

The genetic code for a particular protein, such as Streptolysin O (hereinafter "SLO"), depends upon the sequential grouping of three nucleotides, referred to as a "codon," and the arrangement of such codons in relationship to each other.

A "nucleotide" consists of a nucleoside and one or more phosphate groups. A "nucleoside" consists of a nitrogeneous base linked to a pentose sugar. A "pentose" sugar comprises five carbon atoms. In a molecule of deoxytribonucleic acid, or "DNA", the pentose sugar is "deoxyribose," and the nitrogeneous base can be adenine ("A"), guanine ("G"), thymine ("T") or cytosine ("C"). In a molecule of ribonucleic acid, or "RNA", the pentose sugar is "ribose", and the nitrogeneous bases are the same as DNA, except uracil ("U") replaces thymine. Three types of RNA, messenger RNA, or "mRNA", transfer RNA, or "tRNA", and ribosomal, or "rRNA", translate the genetic information encoded in the DNA into, e.g., a polypeptide or a protein. Thus, genetic information is generally transferred as follows: DNA→RNA→protein.

The sequence of the nitrogenous bases of the DNA molecule encodes the genetic information contained in that molecule. The sugar and phosphate groups of the DNA molecule perform a structural role, forming the backbone of a series of DNA molecules, referred to as a DNA "macromolecule." DNA consists of two complementary strands of nucleotide chains, and these strands are held together by (relatively) weak hydrogen bonds. The bases of each DNA molecule bind to each other: A always bonds with T and C always bonds with G. Thus, the sequence 5'-ATCG-3' of a first strand lies immediately opposite a complementary sequence 3'-TAGC-5' on the other strand. This is referred to as "complementary base pairing." The process of complementary base pairing is referred to as "hybridization" and results in the formation of a stable double stranded DNA macromolecule.

Each codon specifies one amino acid. "Amino acids" are the principal components of proteins, and "proteins" are the essential constituents of all living cells. There are 20 natural amino acids. Because there are four nucleotide bases (A, C, G and T) and three nucleotides per codon, there are 64 possible codons (4³). Accordingly, because there are only 20 natural amino acids, most amino acids are specified by more than one codon. This is referred to as "redundancy" or "degeneracy". For example, the codons GCG, GCA, GCT and GCC all encode for the amino acid Alanine.

The codon ATG (Met amino acid codon) is the normal "start" codon. The codons TAA, TAG and TGA, which do not encode for amino acids, are normal "stop" codons. The formation of mRNA is established based upon the start codon of one strand of the double stranded DNA macromolecule such that the resulting single stranded mRNA will have a nucleotide sequence complementary to the sequence of a single strand of the DNA. When a stop codon is reached by the mRNA along the DNA molecule, translation is stopped.

The regions along the DNA macromolecule which are translated from the mRNA are referred to as "exons" for eukaryotes, and "translated regions" for prokaryotes. "Genes" include exons (eukaryotes) and translated regions (prokaryotes). Thus, genes encode for proteins and/or polypeptides. Mammals, for example, are eukaryotes; bacteria, for example, are prokaryotes.

The natural synthesis of protein takes place over a series of several steps. The first step is the formation of an mRNA macromolecule complementary to the DNA macromolecule, as noted above. Thereafter, tRNA is manufactured; the tRNA provides a complementary codon ("anti-codon") for each codon on the mRNA macromolecule. Thereafter, rRNA will catalyze the assembly of the codon-specific amino acids resulting from the mRNA:tRNA into proteins and/or polypeptides.

### II. Recombinant DNA Technology

Most proteins are produced naturally in extremely small quantities. The advent of recombinant DNA technology has allowed for the production of large quantities of proteins that were previously only available in such small quantities.

The following describes a "typical" genetic manipulation as it might apply to Escherichia coli, a typical bacterial host used for cloning.

In order to isolate, or "clone", a gene, a DNA library is constructed from a DNA sequence (referred to as a "genome") using vectors. A "vector" is a small circular molecule of double-stranded DNA that occurs naturally in bacteria, yeast and mammalian cells. Vectors generally comprise the following characteristics: (i) a DNA sequence encoding a selectable "marker" which assures that the vector will be maintained in an appropriate host cell (e.g., E. coli); (ii) a controllable transcriptional promoter -- by "controllable" is meant that the promoter can be "switched on" by manipulation of, e.g. the environment of the vector; a "promoter" is a region of DNA sequence that when switched on produces large amounts of mRNA from the gene of interest inserted into the vector--different promoters (e.g., lac, trp, tac, etc.) have different rates of mRNA production; (iii) translational control sequences, for example, an appropriately positioned ATG start codon and ribosomal binding site; and (iv) a polylinker; a "polylinker" simplifies the insertion of the gene of interest in the correct orientation within the vector. Vectors can be engineered to provide restriction endonuclease sites on either side of an ATG start codon located on the vector such that the gene of interest can be inserted next to the start codon; this allows for immediate transcription of the gene upon activation of the promoter gene.

A "restriction endonuclease" is an enzyme which cuts the double-stranded DNA at specified sequences of four to eight nucleotides in length, and many restriction endonucleases produce staggered cuts that leave a short, single-stranded tail at the location of the cut. This end is referred to as a "cohesive" or "sticky" end because it can form complementary base pairs with another sticky end. The genome is cleaved (cut-up) by a specified restriction endo-nuclease corresponding to the restriction endo-nuclease used to cut the vector, and the individual pieces of the cleaved genome are inserted into the vector. Randomly cleaving the entire genome of a cell with a specific restriction endo-nuclease is typically referred to as the "shotgun" approach to gene cloning. The shotgun approach can produce an extremely large number of DNA fragments, all of which are inserted into vectors.

The individual pieces of the genome and the vectors, having corresponding sticky ends, are "fused" or "annealed" together to form circular hybrid DNA "plasmids" comprising a portion of the genome and the vector.

The plasmids are then introduced into host cells. There are two types of host cells, "eukaryotic" and "prokaryotic". An example of a eukaryotic host cell is the chinese hamster ovary ("CHO"); an example of a prokaryotic host cell is E. coli bacteria. For purposes of the discussion to follow, attention will focus on prokaryotic host cells.

When the plasmids are introduced into the host cell, these cells are referred to as being "transformed" with the plasmids. As the cells grow and divide, the plasmids will similarly replicate to produce copies of the plasmids containing the DNA fragments. Each transformed cell is referred to as a "genomic DNA clone" and the entire collection of transformed cells containing all of the different DNA fragments is referred to as a "genomic DNA library".

In order to determine which genomic DNA clones contain the DNA sequence capable of being copied into a corresponding mRNA, it is necessary to separate or "screen" the genomic DNA clones. There are several ways to accomplish this task including, for example, the use of radioactive DNA probes or evidence of immuno-reactivity. Screening can be an extremely labor intensive process because, as noted, the shotgun approach by definition leads to the formation of an extensive number of genomic DNA clones, which must be screened to find potential candidates of interest.

### III. Mutations

DNA macromolecules are chemically quite similar to each other. A and G are quite similar in chemical composition, and C, T and U are equally similar. Thus, in a specified sequence, substitutions of an A for a G or a C for a T may occur. When such a substitution occurs within a codon such that the amino acid encoded thereby remains the same, then the substitution can be referred to as a "silent" substitution, i.e., the nucleotides are different but the encoded amino acid is the same. However, other substitutions can alter the amino acid encoded by the codon; when the nucleotide alteration results in a chemically similar amino acid, this is referred to as a "conservative" alteration, while a chemically different amino acid resulting from the alteration is referred to as a "non-conservative" alteration. Non-conservative alterations of amino acids can result in a molecule quite unlike the original protein molecule.

A protein that has had its amino acids altered can be referred to as a "mutant", "mutation" or "variant." Mutations occur naturally and can have positive, negative or neutral consequences on the organism experiencing such a mutation. However, such mutation rates are typically very low, i.e., about 10⁻⁹ to about 10⁻¹⁰ mutations per base replicated.

### IV. Streptolysin O

Streptolysin O ("SLO") has an approximate molecular weight of between about 65,000 and about 70,000 daltons. SLO belongs to a class of oxygen sensitive ("thiol-activated"), cell destroying ("cytolytic") toxin ("cytotoxin") which are produced by gram-positive bacterial species belonging to four different genera (streptococcus, bacillus, clostridium and listeria).

SLO interacts with membrane cholesterol and exerts cytolytic-cytotoxic effects on a broad range of mammalian cells. Additionally, SLO has very potent cardiotoxic properties. One of the toxic and pathogenic properties associated with SLO is its hemolytic activity, i.e. SLO will lyse red blood cells, resulting in the release of hemoglobin. SLO can be lethal to laboratory animals in relatively small doses. Injection of SLO into an animal typically results in its immediate death.

Because SLO is produced by specified bacterial species, when these species "invade" a mammalian host, the SLO released by the bacteria is treated by the host as a foreign protein. SLO, then, is an antigen. "Antigens" are high molecular weight compounds which upon entry into the blood stream of a vertebrate stimulate the transformation of the small lymphocytes of the B-type into lymphoblasts. The lymphoblasts secrete antibodies specific to the antigen stimulator. The antibodies are proteins possessing reactive sites specifically complementary to a reactive feature or site on the stimulating antigen. Antibodies generally have the property of rendering the antigen harmless to the host organism by occupying the immunologically active sites, or "epitopes", on the antigen particles or molecules. Anti-SLO antibodies ("ASO") are therefore produced by the host in response to the secretion of SLO into the host. Approximately 80-85% of individuals with current streptococcal infection or their sequelae (an after effect of a disease or injury) will demonstrate elevated levels of ASO.

Determination of previous and/or current infection by an SLO producing bacteria (S. pyogenes) is possible using immunodiagnostic assaying techniques which, e.g., rely upon the hemolytic properties of SLO and the binding of ASO to SLO. Focusing on hemolytic immunodiagnostic assays for SLO, a patient sample is added to a known amount of SLO derived from a source other than the patient and this mixture is added to a known amount of red blood cells such as, for example, rabbit red blood cells. Because SLO has hemolytic properties, it will lyse these red blood cells. However, when ASO binds to SLO, the hemolytic properties of SLO are neutralized. Thus, if the sample is obtained from a patient having current streptococcal infection or their sequelae, there will be elevated levels of ASO in the sample. Accordingly, if the mixture results in high levels of hemolytic activity, this indicates that there is little, if any, ASO in the serum sample (and hence little, if any, infection from the SLO secreting bacteria) because the known quantity of SLO in the mixture is capable of lysing the known quantity of red blood cells in the mixture. If the mixture does not lead to hemolytic activity, this is indicative of an amount of ASO in the sample sufficient to inactivate the known quantity of SLO in the mixture. Investigators refer to such an amount of ASO as a "titer". Typically, an ASO titer of greater than about 300 International Units/ml is indicative of infection by a bacterial source capable of secreting SLO. Other immunodiagniostic assays for determination of infection by SLO secreting bacteria include nephelometric and turbidimetric protocols. In order to utilize the immunodiagnostic assaying technique outlined above, it is necessary to have access to sufficient SLO to be added to the mixture. One source of SLO is culture broths containing the bacteria *Streptococcus pyogenes* ("S*.* pyogenes"). However, obtaining SLO in this manner is quite difficult and costly: for every liter of the S. pyogenes culture broth, only about 0.5mg of SLO can be expected; the typical media for growing S. pyogenes is expensive; S. pyogenes is a class 2 pathogen; and SLO obtained in this manner contains many other antigenic materials. Additionally, SLO obtained by this procedure tends to be unstable in liquid form. Accordingly, such SLO preparations are most typically supplied as lyophilized powder in vials. Before use, the lyophilized powder must be reconstituted in a suitable solvent. Unfortunately, such reconstituted SLO will rapidly lose its hemolytic activity and therefore it must be used within a brief period after reconstitution or discarded. This has one notable and negative consequence: it is usually impossible to test individual serum samples as soon as they are obtained. Thus, laboratories which conduct ASO assays based upon hemolytic activity typically store the individual samples until a sufficient number are collected to enable economic use of the lyophilized SLO. This can result in an inordinate delay in obtaining test results.

ASO assays which rely upon nephelometric or turbidimetric protocols need significant amounts of purified SLO. Because of the costs associated with obtaining significant quantities of purified SLO from S. pyogenes is expensive, the foregoing hemolytic based assay was the first ASO assay to become commercially available.

Finally, because SLO produced by S. pyogenes is by definition wild-type SLO, it is hemolytically active, and therefore must be handled with extreme caution.

Recombinant DNA techniques for obtaining SLO fusion products offer the benefit of obtaining relatively large quantities of such products. Using such technology, it would be possible to avoid the tedious and cost-ineffective aspects of obtaining SLO from S. pyogenes. In the co-pending application referenced above, SLO derivatives are disclosed. As used herein, the term "SLO derivative" is an SLO fusion product which is soluble, hemolytically active and which is recognized by ASO. SLO derivatives are designated herein as "rSLO". These SLO derivatives are provided in large quantities, are substantially pure, and maintain hemolytic activity.

The gene encoding Streptolysin O (SLO) has been cloned and expressed in *Escherichia coli* (*E. coli*)*.* The complete nucleotide sequence of the cloned SLO gene has been determined and the amino acid sequence of SLO has been deduced from the nucleotide sequence of the gene (Kehoe et al 1987, Infection and Immunity 55:3228-3232). A derivative of Streptolysin O has been disclosed which contains no cysteine amino acids but retains cytolytic activity (GB-A-2 233 977, Kehoe et al).

Another derivative of Streptolysin has been disclosed (EP-A-0 369 825). This derivative is non-toxic, non-cytolytic and retains at least one epitope from wild-type.

It would be beneficial to obtain 'variants" of SLO derivatives obtained from such recombinant DNA techniques. As used herein, the terms "variant", "mutation" or "mutant" are synonymous. SLO variants are designated herein as "mSLO". An mSLO fusion product would provide several benefits, most notably in the manufacturing of immunodiagnostic assays. This is because such an MSLO fusion might be created which would be recognized by ASO antibodies, but would not have associated therewith the hemolytic activity of wild-type SLO.

### SUMMARY OF THE INVENTION

The present invention provides SLO variants. These variants, designated herein as "mSLO", comprise the following characteristics and are broadly defined thereby: (i) recognized by wild-type anti-streptolysin O antibodies (ASO), i.e. comprising at least one epitope characteristic of wild-type Streptolysin O; and (ii) substantially non-hemolytic activity. As used herein, the term "recognized" means capable of being bound by at least one antibody directed against wild-type SLO; the phrase "substantially non-hemolytic activity" means an inability to lyse red blood cells at equivalent titers of wild-type SLO; and "wild-type SLO" is accorded the usual definition associated with such phrase, i.e., SLO that is naturally secreted by a capable bacterial source. "Wild-type SLO", by definition, does not include, e.g., SLO fusion products derived via recombinant DNA techniques.

Another aspect of the invention is directed to a method for making mSLO. The method comprises introducing a soluble, hemolytically active fragment of SLO (e.g., wild type or rSLO) in translational fused or unfused form to a host capable of producing high levels of mutagenic activity, to obtain a mutation fragment candidate; introducing said mutation fragment candidate into an appropriate vector to obtain a plasmid; transfecting a host capable of expressing the mutation fragment candidate with the plasmid to obtain a mutation protein candidate; screening said mutation protein candidate for an absence of hemolysis of red blood cells. Preferably, screening of said candidate for solubility is also conducted. As used herein, the phrase "high levels of mutagenic activity" means greater than about 10⁻⁹ to about 10⁻¹⁰ mutations per base replicated.

Another aspect of the invention includes various kits, such as, for example, test kits for ASO titers, calibrator kits and control kits. A test kit for determining ASO titer comprises mSLO in either liquid or non-liquid form. Most preferably, the mSLO is in liquid form because this allows for ASO titer determination on a per-test sample basis. Calibrator and control kits comprise various concentrations of the mSLO such that calibration and control of ASO titer analysis can be accomplished.

A particularly useful mSLO in accordance with the present disclosure is designated herein as mSLO.3/6 which, in a purified form, has a specific hemolytic activity of 14 hemolytic units ("HU") per mg.

### BRIEF DESCRIPTION OF THE DRAWINGS

Fig. 1 is the nucleic acid sequence of a most preferred embodiment of an SLO variant, designated mSLO.3/6;
Fig. 2 is the amino acid sequence of mSLO.3/6;
Fig. 3 is the nucleic acid sequence of a most preferred embodiment of an SLO derivative, designated rSLO. 3;
Fig. 4 is the amino acid sequence of rSLO.3.
Fig. 5 is the comparative results of a Western blot analysis of supernatant comprising 0.2µl, 2.0µl, 10µl of rSLO.3 and mSLO.3/6,
Fig. 6 is the result of gel electrophoresis of purified mSLO.3/6(A) and rSLO.3(B);
Fig. 7 is the results of titer experiments for hemolytic activity of mSLO.3/6, rS10.3 and commercially available SLO; and

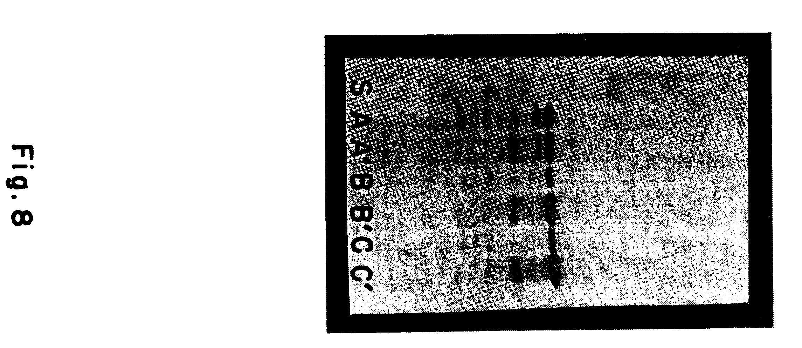

Fig. 8 is the result of Western blot analysis of rSLO.3 and mSLO.3/6 in different vectors.

### DETAILED DESCRIPTION OF PREFERRED EMBODIMENTS

As used in this disclosure, Streptolysin O variants, or "mSLO", comprise the following characteristics and are broadly defined thereby: (i) recognized by wild-type anti-streptolysin O antibodies (ASO); and (ii) substantially non-hemolytic activity. As used herein, the term "recognized" means capable of being bound by at least one antibody directed against wild-type SLO; the phrase "substantially non-hemolytic activity" means an inability to lyse red blood cells at equivalent titers of wild-type SLO; and "wild-type SLO" is accorded the usual definition associated with such phrase, i.e., SLO that is naturally secreted by a bacterial source capable of secreting such protein. Preferably, the mSLO has a percent wild-type SLO specific activity of less than about 1.5%, more preferably less than about 0.5%, and most preferably less than about 0.1%, based upon a wild-type SLO specific activity of 4x10⁵ hemolytic units/mg wild-type SLO. These values are relative; thus, if percent wild-type SLO specific activity is based upon a wild-type SLO specific activity of 1x10⁶ hemolytic units/mg, the above values are decreased by a factor of 2.5 (i.e., 1.5% becomes 0.6%; 0.5% becomes 0.2%; 0.1% becomes 0.04%).

The preceding is detailed because the "specific activity" of wild-type SLO has been described as being as high as about 1x10⁶ hemolytic units/mg, although specific activity of about 4x10⁵ hemolytic units/mg has also been described. Alouf, J. E. "Streptococcal Toxins (Streptolysin O, Streptolysin S, Erythrogenic Toxin)." Pharmac. Ther. II: 661-717 (1980), which is incorporated herein by reference. Accordingly, because the reported "specific activity" of wild type SLO is elusive, the foregoing percentages accomodate this fact.

For convenience, as used herein, the term "vector" means a circular DNA macromolecule comprising at least one restriction site and at least one promoter gene. The term "plasmid" means a vector further comprising a portion of a genome of interest, including, inter alia, a gene. The term "host" means a cell capable of being transfected by a plasmid.

As those in the art appreciate, most vectors are selected with respect to a desired outcome. For example, in a commercial setting, high-level expression of the gene of interest will typically be preferred such that vectors with an appropriate promoter conducive to such expression will be chosen; on the other hand, in a research setting, such high level expression may not be critical such that a vector having translational and transcriptional signals that are under the control of regulatory elements of the host may be appropriate. Accordingly, in selecting a vector appropriate for the desired outcome, it is often useful to concurrently focus on the promoter gene of the vector of interest.

Promoter genes which achieve very high levels of mRNA production include, for example, p_{L}, p_{tac}, and p_{T7}. This list is not intended, nor is it to be construed, as an exhaustive list. Rather these promoters are used as exemplars for purposes of the discussion to follow. Those in the art can readily select an appropriate vector having a desired promoter which can provide equivalent results vis-a-vis the listed promoters.

For example, p_{T7} is used in conjunction with T7 RNA polymerase which synthesizes RNA at a rate several times that of E. coli RNA polymerase and which terminates transcription less frequently than E. coli RNA polymerase. T7 RNA polymerase is highly selective for initiation at its own promoter sequence; accordingly, it does not initiate transcription from any sequences on E. coli DNA. Furthermore, T7 RNA polymerase is resistant to antibiotics such as rifampicin that inhibit E. coli RNA polymerase. Therefore, the addition of rifampicin, for example, to cells that are promoting T7 RNA polymerase results in the exclusive expression of genes under the control of a T7 RNA polymerase promoter, i.e., p_{T7}.

Expression using the T7 RNA polymerase/p_{T7} system relies upon (typically) a two-plasmid system: the first plasmid comprises the gene to be expressed and p_{T7}; the second plasmid comprises the gene for T7 RNA polymerase. The second plasmid, e.g. pGP1-2 (which comprises the gene for T7 RNA poylmerase; see Tabor and Richardson, Proc.Natl.Acad.Sci. U.S.A. 82: 1074-1078(1985)), can either permanently reside in E. coli or can be introduced into E. coli with a specialized phage, such as, e.g., an M13 vector (such as, e.g., mGP1-2, see Tabor and Richardson), or a λ vector (such as, e.g., CE6, see Studier and Moffett, J.Mol.Biol., 189: 113-130(1986)) comprising the T7 RNA polymerase gene.

Typically, the second plasmid comprising the T7 RNA polymerase gene is under the control of a heat inducible E. coli promoter, i.e., by raising the temperature from, e.g., 30°C to 42°C, the heat inducible E. coli promoter is switched on, which will in turn switch on the P_{T7} promoter of the first plasmid, thereby leading to the expression of, e.g., the gene of interest. Thus, when using a T7 RNA polymerase/p_{T7} expression system, the E. coli system comprises a heat-inducible promoter, such as, for example lambda P_{L} with a CI₈₅₇ repressor.

Examples of vectors comprising p_{T7} include, e.g., the pT7 series (pT7-5, pT7-6, and pT7-7, which are derivatives of pT7-1; see Tabor and Richardson, supra.) and the pET series (see Studier et al., Methods Enzymol. 185:60 - 89(1990)).

Another vector system comprises a p_{L} promoter gene. The p_{L} promoter is derived from the λ bacteriophage and is one of the most powerful regulated E. coli promoter. Transcription from p_{L} can be fully repressed and therefore plasmids comprising p_{L} can be stabilized by the λ repressor, cI. This repressor is typically supplied by an E. coli host which comprises an integrated copy of a portion of the λ genome. Such an E. coli host, referred to as an "E. coli lysogen" is characterized as follows: (i) it supplies the λ regulatory proteins cI and N (an anti-termination function); and (ii) it does not provide lytic components that would normally lead to cell lysis. Accordingly, E. coli lysogens transfected with plasmids comprising, e.g., a gene of interest and p_{L}, can be grown initially to high density without expression of the gene and subsequently induced to synthesize the protein under inactivation of the repressor. Examples of p_{L} based vectors are described in, e.g., U.S. Patent No. 4,925,799 ("pAS1"), Shatzman and Rosenberg, "The pAS Vector System and Its Application to Heterologous Gene Expression in Eschericia coli." Heptalogy 7:305-355(1987), and Rosenberg et al., "The Use of pKC30 and its Derivatives for Controlled Expression of Genes." Methods Enzymol 101: 123-139(1983).

The p_{tac} promoter is a hybrid promoter based on the tac and lac promoters. de Boer, et al. "The tac promoter: A functional hybrid derived from the trp and lac promoters." Proc.Natl.Acad.Sci.USA 80:21-25(1983); see also, Amann, et al. "Vectors bearing a hybrid trp-lac promoter useful for regulated expression of cloned genes in Eschericia coli." Gene 25:167-178(1983). Because p_{tac} includes the lac operator region, it can be repressed by E. coli strains that overproduce the lac repressor, and be fully induced by addition of isopropyl β-D-thiogaloctoside (IPTG) thereto.

All of the foregoing references are incorporated herein by reference.

The choice of an appropriate vector/host system is within the realm of the particular needs of the artisan. A most preferred vector is based upon the p_{L} promoter. Table I sets forth a representative (not exclusive) list of suitable vectors and hosts, as well as the sources thereof.

**TABLE I**

| Vector | Host* | Source |
|---|---|---|
| pBTac1 DNA | JM101, JM105, | (1) |
| | JM107, JM109 | |
| | | |
| pBTac2 DNA | JM101, JM105, | (1) |
| | JM107, JM109 | |
| | | |
| pNH8A | D121OPH, D1210 | (2) |
| | | |
| pNH16A | D121OPH, D1210 | (2) |
| | | |
| pNH18A | D121OPH, D1210 | (2) |
| | | |
| pPROK-1 | JM109 | (3) |
| | | |
| pEX2 | N4830-1 | (3) |
| | | |
| pUC19 | JM101, JM105, | (4) |
| | JM107, JM109 | |
| | | |
| p33 | AR120, AR58 | (5) |
| | | |
| p 33 | AR120, AR58 | (5) |
| | | |
| ₚP_{L}-Lamda | N99cI⁺-N4830-1 | (6) |

| | | |
|---|---|---|
| *** = E. COLI CELL** | | |
| **(1) = BOEHRINGER MANNHEIM** | | |
| **(2) = STRATAGENE CLONING SYSTEMS** | | |
| **(3) = CLONETECH LABORATORIES, INC.** | | |
| **(4) = BETHESDA RESEARCH LABS** | | |
| **(5) = SMITHKLINE BECKMAN NOW SMITHKLINE BEECHAM** | | |
| **(6) = PHARMACIA LKB** | | |

For the following examples, the vectors pΔ33 and pBTac2 DNA were utilized in conjunction with the host strains AR120 and JM105, respectively, for the subcloning (initially from pUC19 vector) and expression of mSLO.3/6.

### EXAMPLES

The following Examples directed to preferred embodiments are not intended, nor are they to be construed to be, limitations on the disclosure of the claims to follow.

### Example 1

### Preparation of Partially Digested Genomic Streptolysin O DNA

Genomic DNA was isolated from *Streptococcus pyogenes* (ATCC #10389) using the technique described in Kehoe, M. et al. Infect. Immun., 55:3228-3232 (1987) (hereinafter "Kehoe, 1987"), which is incorporated herein by reference. Approximately lmg of S. pyogenes DNA was obtained using this procedure (925µg).

To 370µl of S. pyogenes DNA (2.5µg/µl) was added 300µl of 10X High Salt Buffer (1.0M NaCl; 100mM tris-hydroxyamino methane-chloride ("TRIS-Cl"), pH7.5; 100mM MgCl₂; and 10mM dithriothreotol ("DTT")), 2310µl of deionized H₂O and 20µl of Bgl II (BRL, Gaithersburg, MD, Cat. # 5213SA), for a final volume of 3000µl. This mixture was maintained at 37°C and incubated overnight.

To this incubated mixture was added 3000µl of Reagent A (250µl phenol, 250µl chloroform, 10µl isoamyl alcohol, 1µl β-mercapthoethanol). This mixture was agitated prior to centrifugation in order to separate the aqueous and the organic layer. The aqueous supernantant was then precipitated with .3M NaOAc and 95% ethanol. The precipitate was then redissolved in 250µl TE (10mM TRIS-Cl, pH 7.5; 1mM EDTA) and 25µl of 10X loading dye (0.2M EDTA; 50% glycerol; 0.25% xylene cyanol; 0.25% bromophenol blue) was added thereto, followed by electrophoresis on 1% agarose gel. The Bgl II partially digested S. pyogenes genomic DNA fragments were then evaluated according to size.

As noted, SLO has an approximate molecular weight of 65,000 to 70,000 daltons. Each amino acid has an approximate molecular weight of 110 daltons, such that (conservatively estimating) a 70,000 dalton protein would be encoded by approximately 636 codons, or 1909 base pairs. Accordingly, the partially digested fragments of between about 2,000 to 2,500 base pairs (i.e., 2.0 to 2.5 Kb), as determined by the aforementioned gel electrophoresis method, were purified. The purified fragments were then resuspended in 150µl of TE. For convenience, these are designated herein as "SLO inserts".

### Example 2

### Preparation of Streptolysin O Containing Plasmids

The vector utilized was pUC19 (BRL, Cat. # 5364SA) cut with Bam HI (BRL, Cat. # 5201SA).

To 1µl of cut pUC19 vector was added 15µl of the SLO inserts, 3µl of 10X ligation buffer (660mM TRIS-Cl, pH 7.5; 50mM magnesium chloride; 10mM DTT; 10mM ATP). A final volume of 30µl was achieved by the addition of 8µl of deionized H₂O. To this mixture was added 2µl of T4 ligase (USB, 5µg/µl); incubation thereof at room temperature proceeded overnight. For convenience, the resulting material is designated as "SLO plasmid candidates".

### Example 3

### Screening of SLO Plasmid Candidates

Host cells E. coli strain JM105 were transformed with the SLO plasmid candidates as follows. A vial containing 300µl of frozen JM105 competent cell was thawed, and 16.0µl of the SLO plasmid candidates was added thereto. This admixture was incubated on ice for 30min, followed by heat shock in a 37°C water bath for 2min. Thereafter, the transfected JM105 solution was added to 2ml of LB medium (10g Bacto-tryptane; 5g Bacto yeast extract; 10g NaCl; 1 liter deionized water; pH 7.5 with sodium hydroxide), followed by shaking (200RPM) for 30min at 37°C. Plating was thereafter accomplished on LB Ampicillin plates, followed by incubation overnight at 37°C.; for convenience, these are designated "SLO transformants".

Screening was accomplished utilizing a unique procedure. Following overnight growth, the colonies were overlaid with 3ml of 2.5% washed rabbit red blood cells in 0.8% agarose in PBS/10mM DTT, which was spread to cover the plates. After 40min of incubation at 37°C, colonies comprising SLO were surrounded by small zones of hemolysis. In order to confirm that these colonies comprised SLO, a 25-mer oiigonucleotide probe derived from nucleotides 670 through 694, inclusive, of the reported DNA sequence of SLO (see Kehoe, 1987) was used as a probe. The probe was prepared with a BioSearch 8600 DNA synthesizer, and labelled with ³²P following the T4 polynucleotide kinase procedure described in Maniatis et al., Molecular Cloning, CSPL (1982), pp. 122-126 (hereinafter "Molecular Cloning").

The blood overlay screening technique proved to be an efficient and accurate method for rapidly screening the SLO expressed by the SLO transformants. Because a property of SLO is the ability thereof to lyse red blood cells, red blood cells from any source can be utilized, i.e., human, mouse, goat, rabbit, etc. Rabbit red blood cells are preferred due to the availability thereof.

An SLO clone that led to the expression of protein which evidenced hemolytic activity and which hybridized with the 25-mer probe was designated "pUC19-SLO-B". For convenience, the non-vector DNA sequence thereof is designated herein as "rSLO-candidates".

### Example 4

### Optimisation of Expression and Determination of Solubility

In order to optimize the expression of rSLO-candidates, timed-digestion of rSLO-candidates using Bal-31 was accomplished. Additionally, and as previously noted, solubility of the expressed protein ab initio, i.e., without further chemical modification once expressed, is of import. This is because non-soluble SLO is by definition inactive. Accordingly, an anlysis was also made to determine if the exprersed protein was soluble, i.e. was located in a supernatant as opposed to a pellet, following centrifugation.

The pUC19-SLO-B was initially cut with BstE II (New England Bio Labs, Cat. # 162, 10 U/µl) as follows. To 20µl of pUC19-SLO-B (2.5µg/µl) was added 40µl of 10X High Salt Buffer, 335µl deionized H₂O, and 5µl of BstE II. This admixture was incubated at 60°C for 2hrs, followed by extraction with 400µl of Reagent A, and precipitation with 44µl of 3M NaOAc (pH 4.8) in 888µl of 95% ethanol. The precipitant was then redissolved in 40µl H₂O. Thereafter, 90µl of H₂O, 20µl of 10X Bal-31 Buffer (120mM CaCl₂; 120mM MgCl₂, 2.0M NaCl; 0.2M TRIS-Cl, pH 8.0; 10mM EDTA), and 50µl of 1mg/ml Bovine Serum Albumin, was admixed with the redissolved precipitant. This was followed by the addition of 10µl of Bal-31 (New England Bio Labs, Cat. # 213, 100 U/ml), for a total of 210µl, followed by incubation at room temperature. To control the effects of Bal-31, 30µl aliquots of the 210µl total solution was removed at 30, 45, 60, 80, 105, 130 and 160min post-Bal-31 addition, and these aliquots were each admixed in 3.3µl of 0.2M EGTA, followed by storage on ice. After preparation and storage of the last aliquot, all seven aliquots were pooled, extracted with 230µl of Reagent A, and precipitated with 23µl of 3M NaOAc in 506µl of 95% ethanol. The precipitate was then redissolved in 75µl of H₂O.

A fill-in reaction followed by the addition of 5µl of 2.5mM dXTP, 10µl of 10X Medium Salt Buffer (500mM NaCl; 100mM TRIS-Cl, pH 7.5; 100mM MgCl₂; 10mM DTT), and 10µl of 100mM of DTT to 75µl of the redissolved precipitant, followed by the addition thereto of 6µl of Klenow polymerase (5 U/µl), and incubation at room temperature for 4hrs. This admixture was extracted with 100µl of Reagent A, precipitation with 11µl of 3M NaOAc in 22µl of 100% ethanol, and resuspension of the precipitate in 40µl of H₂O.

Following the fill-in reaction, 17µl of the resuspended precipitate was admixed with 3µl of a linker comprising a Bam HI sequence (New England Bio Labs, Cat # 1021) and 5µl of 5X linker ligation buffer (250mM TRIS-Cl, pH 7.6; 50mM MgCl₂; 5mM DTT; 5mM ATP; 2.5% (w/v) PEG 8000 (J.T.Baker, Cat. # U222-09)). This was followed by the addition thereto of 2µl of T4 ligase (5 U/µl), and incubation thereof for 6hrs at room temperature. For convenience, the resulting material is referred to as "ca/ew".

E. coli strain JM105 was transformed with ca/ew as described above, followed by overnight growth as described above in Example 3. To determine if the plasmids comprised the Bam HI linker, to 40µl of ca/ew (0.5µg/µl) was added 40µl of 10X Medium Salt Buffer, and 320µl deionized H₂O. To this mixture was added 5µl EcoRI (BRL, Cat # 5202 SA, 10U/µl), followed by incubation at 37°C for 2hrs. In order to ensure that the plasmid was cut, gel electrophoresis (1% agarose gel) was conducted; this resulted in a smear of different sizes, indicating a successful cut. To the cut plasmid was added 8µl of 5M NaCl, followed by 5µl of Bam HI (10 U/µl). This mixture was incubated for 37°C for 2hrs. Determination of the size of the rSLO-candidate sequence subjected to Bal-31 digestion was conducted by gel electrophoresis (1% agarose gel). This resulted in a band of interest at about 1.2 to about 2.0 Kb which comprised rSLO-candidates. Thus, the initial fragments of 2.0 to 2.5 Kb which evidenced hemolytic activity had been significantly decreased in size.

The band which comprised rSLO-candidate was cut from the gel and purified in 15µl of TE such that rSLO-candidate was available for ligation in pUC19 vector previously cut with Bam HI and ECoRI. In order to accomplish such ligation, 10µl of the gel-purified rSLO - candidate was admixed with 4µl of the previously prepared vector, 2µl of 10X ligation buffer, 2µl of 10mM ATP, and 2µl of deionized H₂O. To this admixture was then added 2µl of T4 ligase, followed by incubation at room temperature for 6hrs. E. coli host cell strain JM105 was transformed by these plasmids as above, and active colonies were screened by the red-blood cell overlay method disclosed above. Active colonies were then selected, innoculated in LB Medium/100µg/ml Ampicillin and grown overnight under the conditions described above.

Following overnight growth, the cells were centrifuged for 5min at 8000RPM at 4°C, and the resulting pellet resuspended in 2ml of Reagent B (150mM NaCl; 20mM TRIS, pH 7.0; 1mM EDTA). Thereafter, the resuspended cells were subjected to sonication for 2X 30sec. on ice, followed by centrifugation at 9500RPM for 40min at 4°C using a Beckman JA20.1 centrifuge to obtain the expressed protein.

At this stage, if the rSLO-candidate led to the expression of a soluble protein, that protein would be located in the supernatant. Accordingly, analysis was conducted for the presence of rSLO-candidate in the supernatant using standard Western blot protocols for determination of an antigenically active protein. The results of such Western blot analysis indicated that there was an SLO fusion product in the supernatant which was recognized by horse anti-SLO antibodies. One such fusion product was selected and designated "rSLO.3" For convenience, the DNA sequence leading to the expression of rSLO.3 is also referred to as rSLO.3. High level expression of rSLO.3 was thereafter attempted.

### Example 5

### High Level Expression of rSLO.3

Removal of rSLO.3 from the plasmid comprising pUC19 vector was effectuated as follows. To 15µl of the plasmid comprising rSLO.3 (0.5µg/µl) was added to 40µl of 10X Sma I Buffer (200mM KCl; 100mM TRIS-Cl, pH 8.0; 100mM MgCL₂; 10mM DTT), and 345µl of deionized H₂O. To this mixture was added 5µl Sma I (BRL, Cat # 5228 SA, 10U/µl), followed by incubation at 37°C for 2hrs. In order to ensure that the plasmid was cut, gel electrophoresis (1% agarose gel) was conducted; this resulted in a single band, indicating a successful cut. To the cut plasmid was added 8µl of 5M NaCl, followed by 5µl of Bam HI (10U/µl). This mixture was incubated for 37°C for 2hrs. To ensure that the rSLO.3 sequence was successfully cut from the approximately 2.7Kb pUC 19 vector, gel electrophoresis (1% agarose gel) was conducted. This resulted in two bands, one at about 2.7Kb (the vector), and the other at about 1.4Kb (rSLO.3). This band was cut from the gel and purified in 15µl of deionized H₂O such that rSLO.3 was available for ligation in pΔ33 vector previously cut with Bam HI and Sma I.

To 2µl of above-derived rSLO.3 DNA was added 2µl of the above described vector, 1.5µl of 10X ligation buffer, 1.5µl of 10mM ATP, and 8µl of deionized H₂O. To this admixture was added 2µl of T4 ligase (10 U/µl), followed by incubation for 5hrs at room temperature. Such incubation resulted in plasmids comprising rSLO.3 and pΔ33 vector.

E. coli strain AR120 was transformed with the above-described plasmids in accordance with the procedure outlined for E. coli strain JM105. Thereafter, a DNA mini prep described in Current Protocols in Molecular Biology, Auschel, F.M. et al., Eds, John Wiley & Sons (New York) (1987), Section 1.6., followed by cutting the plasmids with Bam HI and Sal I (BRL, Cat. # 5217 SA) to determine if the plasmids comprised rSLO.3. Those host cells transformed with plasmids comprising rSLO.3 were then subjected to induction via the nalidixic acid protocol. See Mott, J.E. et al "Maximizing gene expression from plasmid vectors containing the λp_{L} promoter: Strategies for overproducing transcription termination factor p." PNAS USA, 82: 88-92(1985), which is incorporated herein by reference. As those in the art appreciate, nalidixic acid, which damages DNA, induces recA protein, a recovery protein for E. coli. A derivative benefit vis-a-vis overexpression is that recA has protease activity, which, inter alia, leads to inactivation of λcI⁺ repressor; this inactivation leads to overexpression by the p_{L} promoter.

Specifically, colonies comprising the transformed AR120 were lifted from the agar plates and innoculated in Superboth (Base - 12g tryptone, 24g yeast extract, 5ml glycerol, 900ml distilled H₂O; Salt (per liter of base) - 1.7g KH₂PO₄, 15.8g K₂HPO₄ (anhydrous) 100ml distilled H₂O) plus 100µg/ml ampicillin at 37°C until the optical density of the medium at A₆₅₀ equalled 0.4. Thereafter, nalidixic acid was added to the innoculated mixture at a final concentration of 60µg/ml and incubated at 37°C for 4hrs. Western blot analysis of the supernatant demonstrated the presence of rSLO.3

### Example 6

### Mutation of rSLO.3

As those in the art appreciate, organisms are equipped with elaborate mechanisms that keep DNA mutation rates low, from a statistical point of view. There are at least three steps required to prevent mutations from occurring during DNA replication: (i) selection of a nucleotide complementary to the template (i.e. the DNA macromolecule being replicated) by DNA polymerase (an enzyme which catalyzes the addition of a nucleotide to the 3' end of a DNA chain); (ii) removal of a noncomplementary base by an "editing nuclease" (i.e., an enzyme capable of degrading, e.g., a nucleotide); and (iii) correction of a misincorporated nucleotide.

As is further appreciated by the skilled artisan, "mutators" are a special class of mutations that render various genes unstable. Bacteria mutator strains, for example, E. coli mutator strains, have been utilized to investigate the mechanisms by which organisms control their mutation rates. Accordingly, identification of specific genes within a strain of, e.g., E. coli, having mutations, allows for investigation of mutator activity.

The dam, mutS, mutR, mutL, and uvrD genes are involved in the process of mismatch repair; accordingly, E. coli strains which include defective versions of these genes exhibit moderately high mutator activity. Some E. coli mutator genes cause specific changes in the DNA sequence being replicated. For example, the mutS3 mutator strain of E. coli produces bi-directional transitions and is apparently sensitive to base sequence, in that it mutates one A:T base pair but does not mutate a second A:T base pair located less than 50 nucleotides away. The mutT mutator strain of E. coli is unique in displaying a strict specificity, i.e., only A:T → C:G transitions are induced. The mutD mutator strain of E. coli is also notable because it results in mutation frequencies of 10³ to 10⁵ times that of wild-type E. coli. Transversions of A:T ↔ G:T, transitions of A:T ↔ T:A, and A:T → C:G substitutions are associated with the mutD strain. While the tendencies of certain mutator strains are known, predicting exactly when or where along the DNA macromolecule a mutation will take place cannot be reasonably predicted.

In an effort to effectuate a mutation of rSLO.3 (having as a desired objective obtaining a mutated form thereof), the E. coli mutator strains mut D and mutT were utilized. A most preferred mutator strain is mutD. Vials containing 300µl each of frozen competent cell mutD and mutT strains were thawed, and 1µl of the plasmids from Example 4 was added thereto. This admixture was incubated on ice for 30 min., followed by heat shock in a 37°C water bath for 2 min. Thereafter, the transformed mutD and mutT host cells were added to 2ml of LB medium, followed by shaking (200RPM) for 30 min. at 37°C. Innoculation was accomplished as follows: to the incubated mixture was added 10ml of LB Medium/100µg/ml Ampicillin, followed by overnight growth at 37°C. Innoculation was repeated 5 (five) times, followed by the DNA mini-prep procedure referenced above. This resulted in plasmids comprising rSLO.3 mutation sequence candidates.

E. coli strain JM105 was thereafter transformed with these plasmids as described above, followed by plating and overnight growth as described above. Screening was accomplished using the unique blood-overlay protocol disclosed above. Most of the resulting colonies were active (i.e. non-mutants); however, a few of the resulting colonies did not lead to lyses of the red blood cells; these are referred to herein as mSLO-candidates. The colonies comprising mSLO-candidates were picked for determination of the immunoactivity thereof. This was accomplished using standard Western blot protocols (horse ASO). One of the mSLO-candidates which evidenced immuno-reactivity with horse ASO was selected for high level expression.

### Example 7

### High Level Expression of mSLO

Having cloned a gene for mSLO, high level expression thereof using a p_{L} based vector was accomplished. The vector was pΔ33; the E. coli host strain was AR120.

Removal of mSLO from the plasmid comprising pUC19 vector was effectuated as follows. To 15µl of the plasmid comprising mSLO (0.5µg/µl) was added 40µl of 10X Sma I Buffer, and 345µl of deionized H₂O. To this mixture was added 5µl Sma I, followed by incubation at 37°C for 2hrs. In order to ensure that the plasmid was cut, gel electrophoresis (1% agarose gel) was conducted; this resulted in a single band, indicating a successful cut. To the cut plasmid was added 8µl of 5M NaCl, followed by 5µl of Bam HI (10U/µl). This mixture was incubated for 37°C for 2hrs. To ensure that the mSLO sequence was successfully cut from the approximately 2.7Kb pUC 19 vector, gel electrophoresis (1% agarose gel) was conducted. This resulted in two bands, one at about 2.7Kb (the vector), and the other at about 1.4Kb. The DNA of within this band is referred to herein as "mSLO.3/6" and protein expressed by this DNA is also referred to as mSLO.3/6. This band was cut from the gel and purified in 15µl of deionized H₂O such that mSLO.3/6 was available for ligation in pΔ33 vector previously cut with Bam HI and Sma I.

To 2µl of above-derived mSLO.3/6 was added 2µl of the above described vector, 1.5µl of 10X ligation buffer, 1.5µl of 10mM ATP, and 8µl of deionized H₂O. To this admixture was added 2µl of T4 ligase (10 U/µl), followed by incubation for 5hrs at room temperature. Such incubation resulted in plasmids comprising mSLO.3/6 and pΔ33 vector.

E. coli strain AR120 was transformed by the above-described plasmids in accordance with the procedure outlined for E. coli strain JM105. Thereafter, a DNA mini prep as referenced above was performed, followed by cutting the plasmids with Bam HI and Sal I (BRL, Cat. # 5217 SA) to determine if the plasmids comprised mSLO.3/6. Those host cells transformed with plasmids comprising mSLO.3/6 were then subjected to the nalidixic acid and innoculation protocols described above. As with rSLO.3, Western blot analysis of the supernatant demonstrated the presence of mSLO.3/6.

### Example 8

### Specific Activity of mSLO.3/6

Protein concentration and specific activity of non-purified mSLO.3/6 obtained from Example 7 was determined and compared with the protein concentration and specific activity of non-purified rSLO.3 immediately following nalidixic acid induction of each.

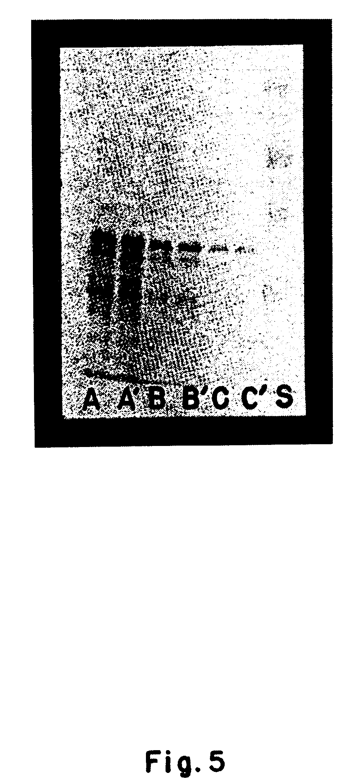

Protein concentrations for both the rSLO.3 and mSLO.3/6 crude extracts were derived using the BioRad Protein Assay method (Coomassie Blue G-250). Nalidixic acid induced proteins mixtures were centrifuged at 8000RPM for 5min at 4°C and the pellets resuspended in 500µl sonication buffer (40mM TRIS, pH 7.5; 1mM EDTA; 1mM DTT; 200 mM NaCl). The resuspended pellets were then sonicated for 2X 30sec on ice, followed by centrifugation at 12,000RPM for 40min at 4°C. Thereafter, 5µl of the resuspended rSLO.3 and mSLO.3/6 mixtures were analyzed for protein concentration (OD reading at A₅₉₅), and the protein concentration of each was determined to be 4.6µg/µl. Western blot analysis was conducted for each sample; the gel pattens for .2µg (C,C'), 2µg (B,B'), and 10µg (A,A') of each protein (A,B,C=mSLO.3/6; A',B',C'=rSLO.3; S=standard marker) were identical, as is evident from Figure 5.

Specific activity comparisons were determined by serial dilutions of the above described crude extracts and addition thereto of washed rabbit red blood cells ("RRBC"), followed by spectrophotometric reading (OD reading at A₅₄₁). 5ml of fresh rabbit blood was washed 2X with 45ml of PBS including 10mM DTT, followed by centrifugation at 2000RPM for 5min at 4°C. Thereafter, 1.125ml of the washed rabbit red blood cells ("RRBC") were drawn from the bottom of the tube and 48.875 of PBS/10mM DTT was added thereto. This resulted in a solution comprising 2.25% RRBC. For the hemolytic assays, 500µl of the 2.25% RRBC was added to 500µl of 1:2 serially diluted rSLO.3 and mSLO.3/6 in PBS/10mM DTT, followed by incubation at 37° for 30min.

These serial dilutions were spectrophotometrically analyzed (OD readings at A₅₄₁). This. analysis indicated that .2µl of the diluted rSLO.3 crude extract caused 50% hemolysis of the RRBC; .2µl of the diluted extract is equivalent to 2µl of the extract itself. Accordingly, the rSLO.3 crude extract evidenced one hemolytic unit ("HU") per two microliters, or 500 HU/ml. The mSLO.3/6 crude extract had substantially no hemolytic activity, i.e, 100µl of the resuspended mSLO.3/6 culture caused less than 6% hemolysis of the RRBC, or .2 HU/ml. Accordingly, there is 2,700 times less hemolytic activity associated with mSLO.3/6 compared to rSLO.3.

As noted, the protein concentration of the crude extracts was determined to be 4.6mg/ml. Accordingly, the specific activity of rSLO.3 derived from the pΔ33-AR120 expression system was 108.7HU/mg, while the specific hemolytic activity of mSLO.3/6 derived from this system was .04HU/mg. It is noted that because these are values for a crude (i.e. non-purified) extract, these values are predicated upon total protein concentration of the extract. For a purified extract, the specific activity values increase.

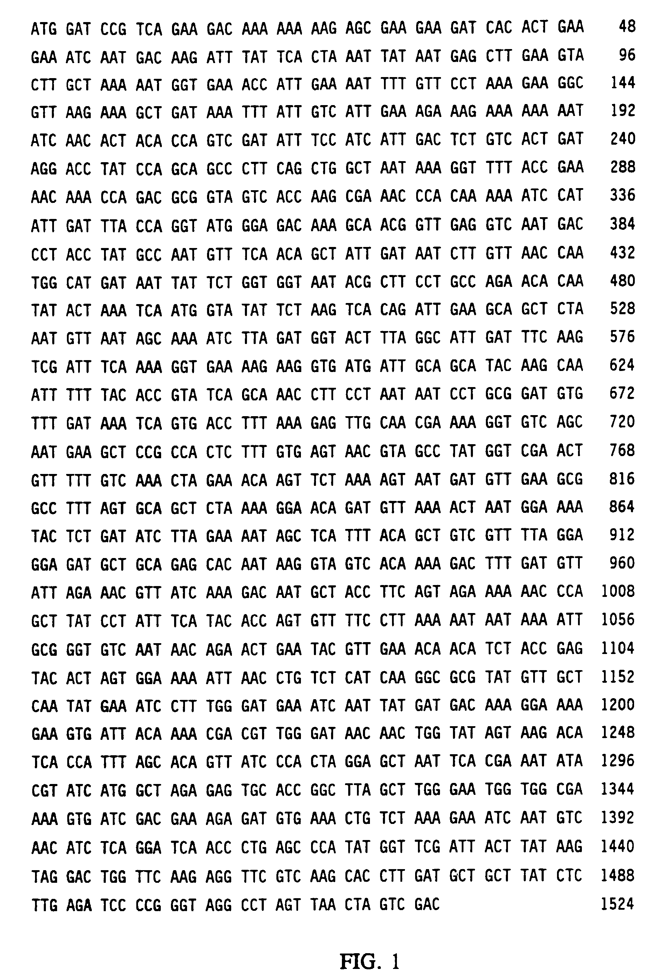

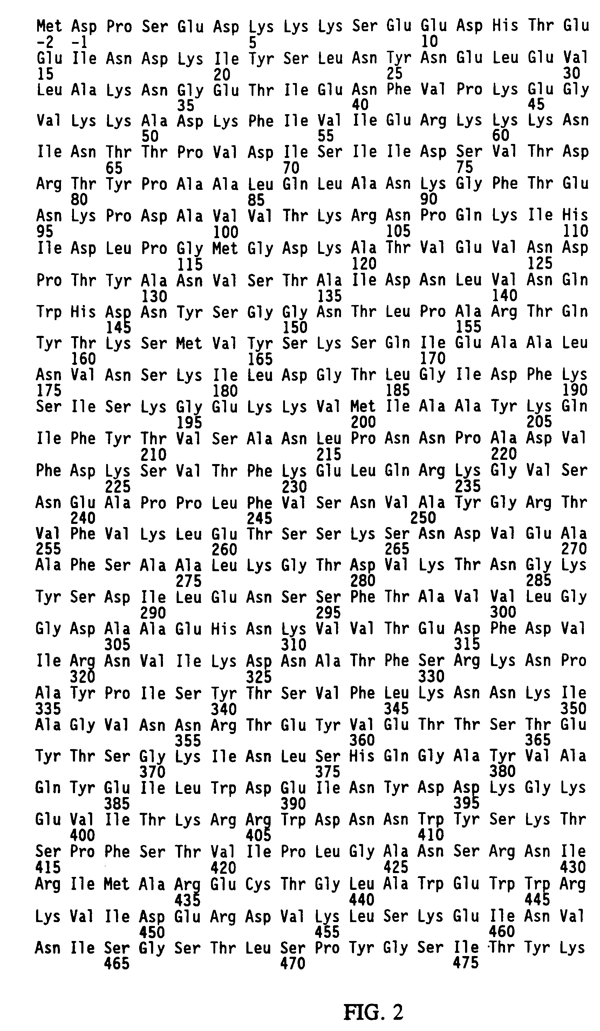

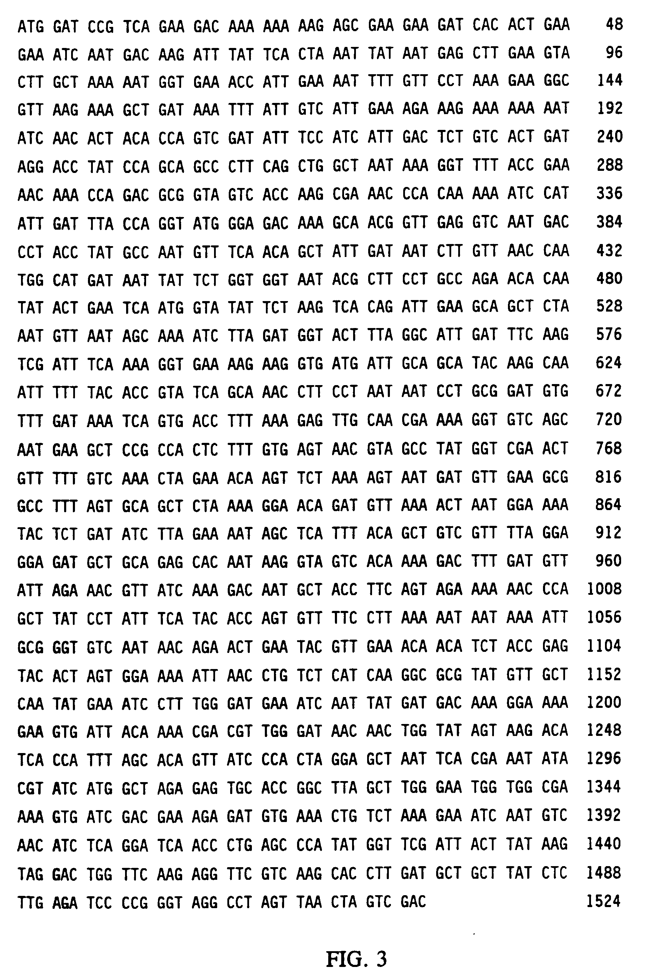

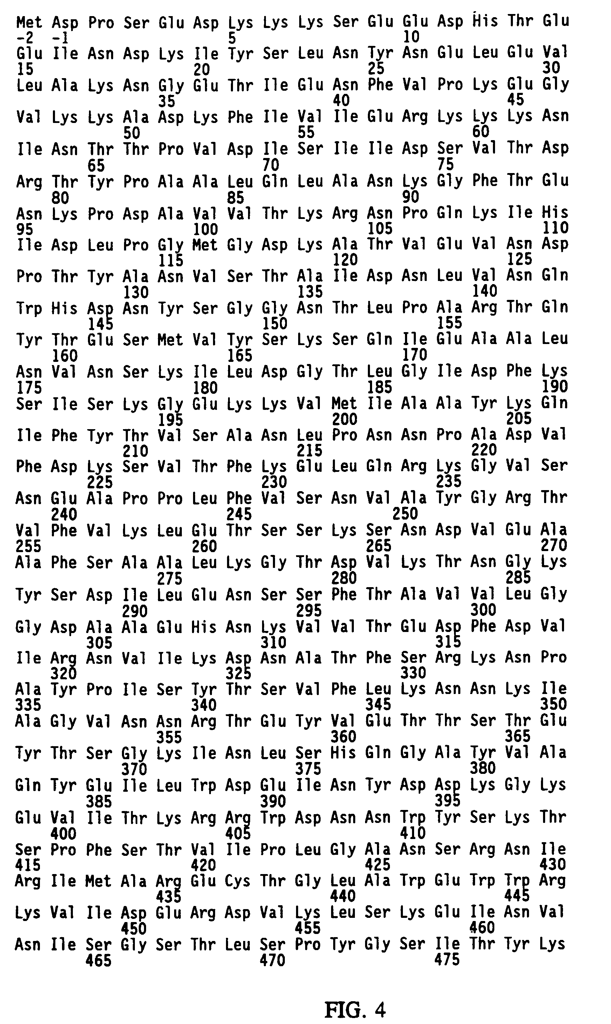

The DNA and amino acid sequences of mSLO.3/6 was thereafter determined (Lark Sequencing Technologies, Houston TX), and are presented in Figures 1 and 2, respectively. In Figure 1, the first codon (ATG) was donated by the pΔ33 vector, and the second codon (GAT) was from the Bam Hl linker. For comparative purposes, the DNA and amino acid sequencing of rSLO.3 are presented in Figures 3 and 4.

There is a single codon difference between the sequences of rSLO.3 and m SLO.3/6 at codon 487. In rSLO.3, this codon is GAA (Glu amino acid)-while in mSLO.3/6, this codon is AAA (Lys amino acid).

### Example 9

### Recovery of mSLO.3/6

The following procedure is for approximately 200 grams of transformed host cells (i.e., approximately 6 grams total protein).

Transfected host cells were resuspended in 200mls of Reagent C (40mM TRIS, pH 7.5; 1mM EDTA; 0.1% 2-mercaptoethanol), followed by the addition of 100mM PMSF. Thereafter, the cells were disrupted by sonication, followed by the addition of 4ml of 100mM PMSF. This admixture was centrifuged for 30min at 4°C at 15,000RPM.

The resulting supernatant was removed and saved; 200ml of Reagent C was added to the pellet, followed by the addition of 4mls of 100mM PMSF. The resuspended pellet was then sonicated, followed by centrifugation as above. The resulting supernatant was then removed and pooled with the previous supernatant, and the pH thereof was adjusted to 7.0 with NaOH.

To the final volume of supernatant was slowly added (with stirring at room temperature) Polymin P (Aldrich Chemicals) to a final concentration of 0.75%. This admixture was then centrifuged for 30min at room temperature at 10,000 RPM, followed by retrieval of the supernatant. Solid sodium sulfate was slowly added with stirring to 80% saturation of the supernatant.

Thereafter, the admixture was stirred for 2hrs at 4°C, followed by centrigation for 30min at 4°C at 15,000 RPM. The pellet was then retrieved and resuspended in 400mls of saturated ammonium sulfate, pH 7.0. The admixture was then centrifuged for 30min at 4°C at 10,000 RPM, followed by retrieval of the pellet and resuspension thereof in 200mls in Reagent D (20mM TRIS, pH 7; 1mM EDTA; 0.1% 2-mercaptoethanol).

The resuspended pellet was then dialyzed against 2 liters of Reagent D, with 4 changes, at 4°C. Sufficient room was left in the dialysis bag in that the volume of the sample increases. Following dialysis, the pH of the sample was checked, and adjusted to 7.0 with NaOH.

The sample was then loaded onto a Pharmacia Fast Flow S-Sepharose column equilibrated in Reagent D. A 400ml bed volume was found to be sufficient to remove the mSLO.3/6 from the sample. The flow through, comprising E. coli proteins, was collected and discarded, and the column was washed with approximately 1 liter of Reagent D.

The mSLO.3/6 was eluted with 2 x 1 liter 0.0 to 0.4M NaCl gradient in Buffer B. The fractions were analyzed by SDS acrylamide gel (9%), and fractions with high amounts of mSLO.3/6 were pooled. Approximately 250ml of pooled mSLO.3/6 was recovered.

Using the above procedure, approximately 60% of the original total protein (i.e. approximately 0.36 grams) was mSLO.3/6, which can be stored at 4°C until needed.

### Example 10

### Purification of mSLO.3/6

Purification of mSLO.3/6 was accomplished to a purity of at least 80% using the following protocol.

Approximately 600g of frozen cell paste derived in accordance with the protocol described in Example 9 was thawed (37°C), resuspended in 3 liters of cold lysis buffer (40mM TRIS-Cl, pH 7.0; 1mm EDTA; .1% 2-mercaptoethanol; 2M Nacl; 4°C) and sonicated for 60min at 4°-10°C with a Heat Systems Ultrasonics Continuous Flow sonicator (Farmingdale, N.Y., No. W-385). Thereafter, the material was centrifuged on a Beckman JA10 centrifuge at 9500RPM for 40min at 20° to 26°C. Approximately 3 liters of supernatant was retrieved.

To the supernatant was added at 12.5% stock solution of Polymin P precipitant (Aldrich, Milwaukee, Wis.) to a final concentration of between 0.2 to 0.3%. The solution was then stirred for lhr at room temperature and the precipitate discarded. The pH of the liquid portion was then adjusted to 7.0 with NaOH. This liquid was then permitted to stand overnight at room temperature.

Thereafter, the solution was centrifuged as above, and a clear supernatant retrieved. The supernatant was then loaded onto a 1 liter phenylsepharose HIC column (Pharmacia, Piscataway, N.J.) at 2ml/min. at room temperature. Thereafter, the column was washed with an elution buffer (20mM TRIS-Cl, pH 7.0; 1mM EDTA; 0.1% BME) at 7ml/min. Fractions were monitored by SDS-PAGE electrophoresis using the Pharmacia Phast-Page™ System. Protein concentration was determined with the BioRad Protein Assay Kit. Fractions containing protein were then pooled.

The pooled fractions was then loaded onto a 1 liter Blue Affinity Column (BioRad, Richmond, California) at 2ml/mm at room temperature, followed by washing using the elution buffer described above at 2ml/min. at room temperature for two column volumes.

Elution of bound protein was accomplished using an NaCL density gradient of 0.0 to 0.8M, pH 7.0. Fractions were monitored with the Phast-PAGE System and protein concentration determined with the BioRad Protein Assay Kit. A single peak was obtained at 0.3-0.4M on the NaCL density gradient.

Purity of the eluted mSLO.3/6 was evaluated using a Beckman DU 7500 spectrophotometer, based upon analysis of major band homogeneity obtained from gel electrophoresis (12% SDS-polyacrylamide) of six different amounts of the eluted mSLO.3/6 (16, 8, 4, 2, 1, .5µg mSLO.3/6). The evaluated purity of mSLO.3/6 (and rSLO.3 for comparative purposes) based upon major band homogeneity is set forth in Table 2:

**Table 2**

| mSLO.3/6 and rSLO.3 (µg) | Percent Homogeneity | |
|---|---|---|
| | mSLO.3/6 | rSLO.3 |
| 0.5 | >99.0% | >99.0% |
| 1.0 | >99.0% | >99.0% |
| 2.0 | >99.0% | 94.4% |
| 4.0 | 86.8% | 82.4% |
| 8.0 | 85.1% | 81.4% |
| 16.0 | 81.9% | 80.1% |

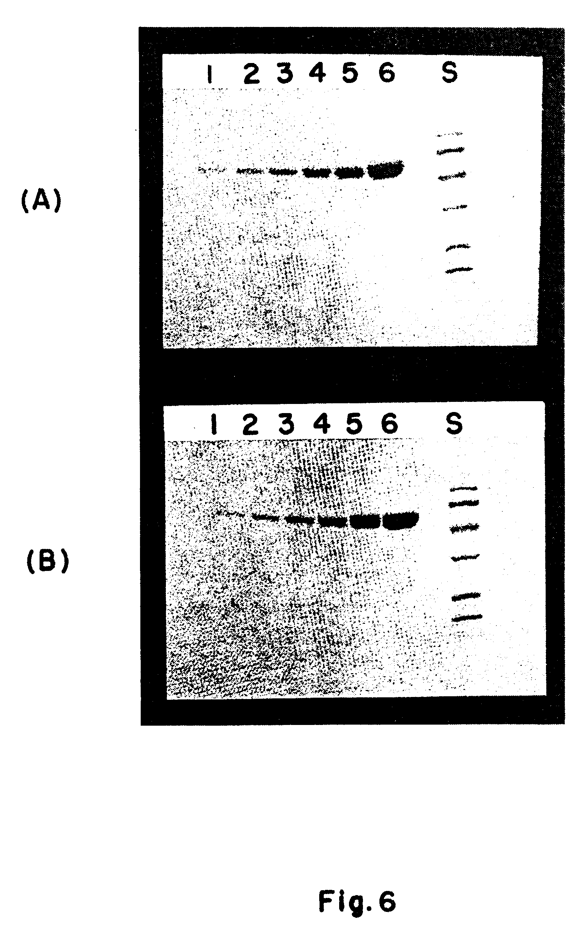

The gel electrophoresis results are presented in Figure 6 where A is mSLO.3/6 and B is rSLO.3.

### Example 11

### Specifc Hemolytic Activity (Titer) of Purified mSLO.3/6

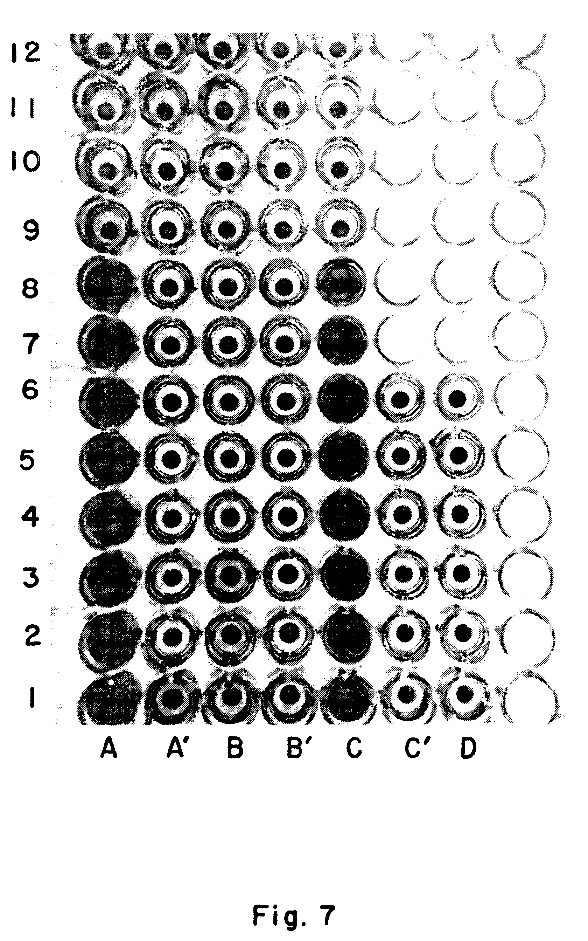

Figure 7 provides a photograph of a 96-well microtiter plate. The microtiter wells evidence the results of an analysis of the hemolytic activity (by titer) of rSLO.3; a commercially available wild-type version of SLO (Sigma, Product No. S-5265); and mSLO.3/6.

For the hemolytic assay titers, 0.154g DTT was added to 1 titer of PBS. Fresh rabbit red blood cells (2.5%) were washed with PBS, followed by centrifugation at 2000RPM for 20min at 4°C; this protocol was followed three times. Initial dilution of samples was 1:50 in the aforementioned PBS-DTT solution based upon 500µl PBS-DTT solution to 10µl of the sample; a 1:2 dilution series was followed for 12 dilutions.

Referencing Figure 7, in well Al, 200µl of diluted rSLO.3 was added thereto; in B1, 200µl of diluted mSLO.3/6; in C1, 200µl of diluted Sigma SLO. In the remaining wells, 100µl of PBS-DTT solution was added thereto. Thereafter, 100µl of the solution from A1 was pipetted into A2; this procedure was repeated such that each well had 100µl of solution mixture. Thereafter, using an 8 channel pipettor, 100µl of the washed rabbit red blood cells were added to each well, followed by gently shaking and incubatim at room temperature for 20 minutes. Thereafter, the plate was centrifuged at 500RPM in a Beckman J4.2 centrifuge for 5min at 4°C.

Dilutions were as follows as represented in Table 3 for the microtiter wells shown in Figure 7:

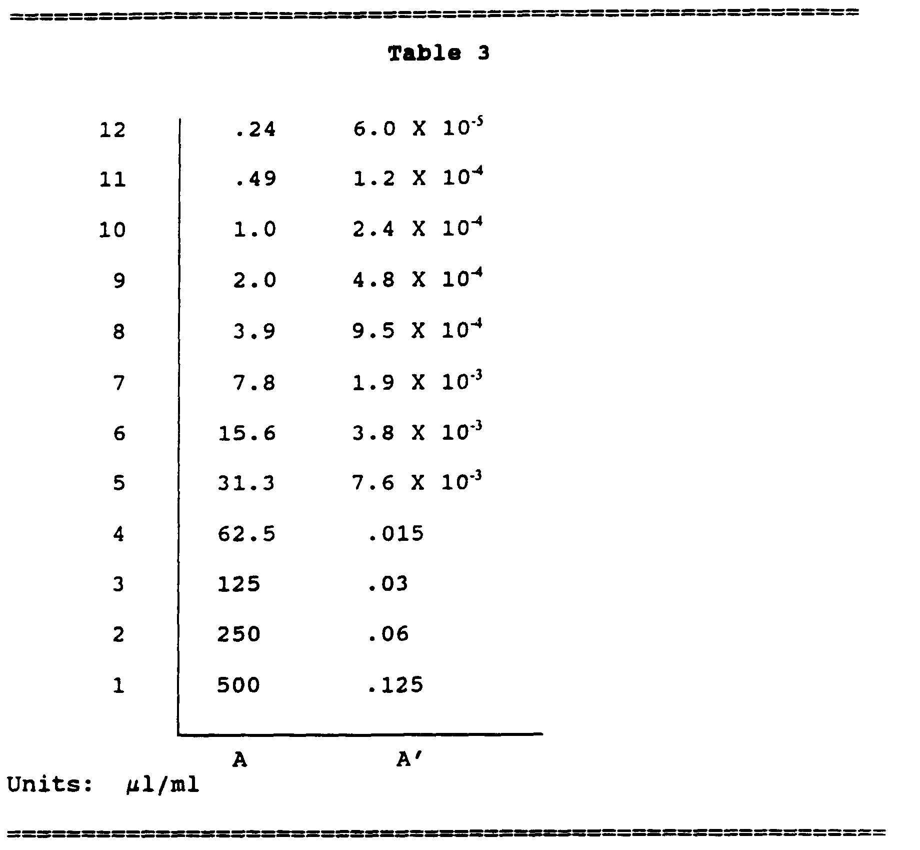

In Figure 7, A,A' is rSLO.3, B,B' is mSLO.3/6, C,C' is Sigma SLO, and D is a negative control (no SLO). Dilutions for B,B, C,C' and D were identical to A,A' except that Sigma SLO was not diluted beyond 1.0 X 10⁻³ µl/ml and the negative control did not extend past D6.

Plates were read by determining which well in the dilution range did not evidence lysis of the red blood cells. If lysis occurs, then the well is red; the lack of lysis is indicated by the pellet of red blood cells located at the bottom of each well (i.e. the "center" of each well).

As is evident from Figure 7, no lysis occurred at any titer for msLO.3/6, while lysis occurred at approximately the same titer for both rSLO.3 and Sigma SLO. Accordingly, mSLO.3/6 evidences substantially no hemolytic activity at any equivalent titer of either the commercially available SLO or rSLO.3.

For determination of hemolytic activity, the concentration of purified mSLO.3/6 was determined (1.2mg/ml). A 1:16.7 titer of mSLO.3/6 was required to obtain greater than 50% lysis of 2.5% RRBC. Accordingly, the specific hemolytic activity of purified mSLO.3/6 is 14HU/mg (16.7 ö 1.2). For comparative purposes, the hemolytic activity of rSLO.3 was similarly determined. A 1:25,600 titer of a .7mg/ml concentration of purified rSLO.3 was required to obtain greater than 50% lysis of 2.5% RRBC. Accordingly, the specific hemolytic activity of purified rSLO.3 is 3.6 x 10⁴HU/mg. This evidences a 2,600 fold difference in specific activity which correlates with the 2,700 fold difference obtained from the crude (non-purified) extracts of Example 8.

### Example 12

### Subcloning of mSLO.3/6

Having obtained, verified and sequenced mSLO.3/6, subcloning and expression thereof using another expression/vector system was initiated. The vector, pBTac 2 DNA (Boehringer Mannheim, Cat. No. 1081381, 10µg) was cut with Hind III (BRL, Cat. No. 52075A, 10 U/ml) by admixing 30µl of pBTac2 DNA (1µg/µl), 30µl of 10X Medium Salt Buffer, 240µl deionized H₂O, followed by addition thereto of 5µl of Hind III (BRL, Cat. # 5207 SA, 10U/µl). This admixture was incubated for 2hrs at 37°C. Thereafter, the admixture was analyzed by agarose electrophoresis (1% agarose gel) to determine if the vector had been successfully cut; a single band indicated that the cut had been successful.

To the 305µl admixture was added 300µl of Reagent A. This admixture was then centrifuged for 5min at 12,000RPM on a Beckman microcentrifuge, followed by retrieval of the upper liquid layer. To this liquid layer was added 33µl of 3M NaOAc (pH 4.8) and 660µl of ethanol, followed by precipitation overnight at -20°C. This was followed by centrifugation for 10min at 12,000RPM on a Beckman microcentrifuge. The pellet was retrieved and dried by air. The dried pellet was then resuspended in 150µl of deionized water.

In order to blunt (fill-in) one end of the Hind III cut vector, the 150µl solution comprising the resuspended pellet was admixed with 10µl of 20X dNTP (2.5mM), 20µl of 10X MSB and 20µl of 100mM DTT. This was followed by the addition of 4µl of Klenow polymerase (New England Biolabs, Cat. No. 210, 5 U/ml) and incubation at room temperature for 7hrs. Thereafter, 300µl of Reagent A was added to the incubated mixture, followed by centrifugation for 5min at 12,000RPM. The upper liquid layer was retrieved and precipitated as above. The dried pellet was then resuspended in 30µl of deionized H₂O. For convenience, the filled-in, Hind III cut vector is referred to as "vec.rb".

Thereafter, vec.rb was cut with Bam HI (BRL, Cat No. 5201 SA, 10 U/µl). To 30µl of vec.rb was added 30µl of 10X High Salt Buffer and 240µl of deionized H₂O. To this admixture was added 5µl of Bam HI, followed by incubation for 2hrs at 37°C. To the incubated mixture was added 300µl of Reagent A, followed by centrifugation as above. The upper liquid layer was retrieved and precipitated as above. The dried pellet was then resuspended in 20µl of deionized H₂O. The resuspended pellet comprised Hind III cut, filled-in, Bam HI cut pBTac2 DNA.

The mSLO.3/6 removed from the plasmid described above as follows. To 40µl of the plasmid comprising mSLO.3/6 (1µg/1µl) was added 10X SmaI Buffer, and 320µl deionized H₂O. To this mixture was added 5µl Sma I (10U/µl), followed by incubation at 37°C for 2hrs. In order to ensure that the plasmid was cut, gel electrophoresis (1% agarose gel) was conducted; this resulted in a single band, indicating a successful cut. To the cut plasmid was added 8µl of 5M NaCl, followed by 5µl of Bam HI (10 U/µl). This mixture was incubated for 37°C for 2hrs. To ensure that the mSLO.3/6 sequence was successfully cut from the approximately 6.3Kb pΔ33 vector, gel electrophoresis (1% agarose gel) was conducted. This resulted in two bands, one at about 6.3Kb (the vector), and the other at about 1.4kB (mSLO.3/6). The 1.4Kb band was cut from the gel and purified in 20µl of deionized H₂O such that mSLO.3/6 was available for ligation in the prepared pBTac2 vector.

To 3µl of the vector was added 2µl of mSLO.3/6, 1.5µl of 10X Ligation Buffer (0.66M TRIS-Cl (pH 7.5), 50mM MgCl₂, 50mM DTT, 10mM ATP), 1.5µl of 10mM ATP and 7µl of deionized H₂O. Thereafter, 1.5µl of T4 Ligase was added thereto, followed by incubation overnight at room temperature. For convenience, this mixture is referred to as the "subcloneₘ".

E. coli strain JM105 was transfected with subcloneₘ as follows. A vial containing 300µl of frozen JM105 competent cell was thawed, and 8.0µl of subcloneₘ was added thereto. This admixture was incubated on ice for 30min, followed by heat shock in a 37°C water bath for 2min. Thereafter, the transfected JM105 solution was added to 2ml of LB medium (10g Bacto-tryptane; 5g Bacto yeast extract; 10g NaCl; 11 deionized water; pH 7.5 with sodium hydroxide), followed by shaking (200RPM) for 30min at 37°C. Plating was thereafter accomplished on LB Ampicillin plates, followed by incubation overnight at 37°C.

Because the resulting colonies comprised mSLO.3/6, the blood overlay screening protocol could not be utilized (i.e., a non-viable colony could also evidence non-hemolytic activity). Accordingly, screening was accomplished using hybridization probes obtained from pUC19-SLO-B cut with BstE II and EcoRV (approximately a 1.1Kb probe), labelled by the random primer labelling method described in Anal. Biochem., 132 6-13(1983).

In order to screen these colonies, pure nitrocellulose transfer membranes (Schleicher & Schuell, Keene, NH, No. 20440; pore size: 0.45µm) were placed onto the plates, and colonies were lifted therefrom. The membranes were subjected to a denaturing buffer (0.5M NaOH; 1.5M NaCl) for 5min at room temperature. This was followed by addition of a neutralizing buffer (1.5M NaCl; 1M TRIS, pH 8.0). The membranes were then baked for 2hrs at 80°C, followed by addition thereto of 12ml of hybridization buffer (5X SSPE; 50% deionized formamide; 5X Denhardts; 100µg/ml denatured salmon testes DNA - see Molecular Cloning) and prehybidized for 1hr at 37°C. Thereafter, the above-described probes were added therto, and hybridized overnight at 37°C. This was followed by three washings (10min each) with 10mM KPO₄, pH 7.0, 1mM EDTA. The membranes were then exposed to x-ray film overnight at - 70°C. Areas indicative of successful hybridization were then used to obtain colonies comprising the mSLO.3/6 subclones.

Screened colonies comprising mSLO.3/6 subclones were innoculted in 12ml of Superbroth-ampicillin broth. Induction was accomplished by the addition of isoprpyl-β-D-thiogalactopyranoside ("IPTG"), at a final concentration of 1mM, to the culture broth when the culture broth had an OD₆₀₀ reading of 0.7. 12ml of the resulting solution was centrifuged at 8000RPM for 10min at 4°C and the resulting pellet resuspended in 1.2ml of PBS/10mM DTT. The resuspended pellet was sonicated for 1.5min; the protein concentration of the sonicated extract was determined using the BioRad Protein Assay protocol described above. For the extract comprising mSLO.3/6, the protein concentration was 13mg/ml. This data was used to determine the specific hemolytic activity of the sonicated extract by titer based upon the 50% lyses of 2.5% washed rabbit red blood cell protocol described above.

For purposes of comparing the specific hemolytic activity of mSLO.3/6 with that of rSLO.3 using the pBTac2 - JM105 expression system, rSLO.3 was also obtained using this expression system following the specific procedure described above (derived protein concentration of extract comprising rSLO.3 - 9.3mg/ml). Results are provided in Table 3:

**Table 3**

| **Sonicated Extract (µg)** | | **>50% Lysis (+)** | **Hemolytic Units per mg extract** |
|---|---|---|---|
| | | **<50% Lysis (-)** | |
| | 260. | (-) | |
| | 520. | (-) | |
| **mSLO.3/6** | 1040. | (-) | |
| | 2080 | (-) | 4.8 x 10^{-1*} |
| | | | |
| | 0.186 | (-) | |
| | 0.372 | (+) | 2.69 x 10³ |
| **rSLO.3** | 0.744 | (+) | |
| | 1.116 | (+) | |
| | 4.650 | (+) | |

| | | | |
|---|---|---|---|
| * - Estimated based upon 25% lysis at endpoint which contains 2080µg extract per ml of buffer | | | |

The foregoing data indicates that for the pBTac2 - JM105 expression system, the crude extract comprising mSLO.3/6 is approximately 5,600 times less hemolytically active than the crude extract comprising rSLO.3.

### Example 12

### Comparative Analysis of Expression Systems

In an effort to compare the relative expression sytems vis-a-vis the expression of rSLO.3 and mSLO.3/6, Western blot analysis was conducted on the pellets and supernatants for the pΔ33 vector comprsing rSLO.3 and pBTac2 vectors comprising rSLO.3 and mSLO.3/6. Identical protocols were followed for side-by-side comparative studies; each transformed host was innoculated in Superbroth, and respective induction strategies were used, i.e., naladixic acid for pΔ33, and IPTG for pBTac2. Figure 9 provides the results of the Western blots of the crude extracts from the various materials indicated (S=standard marker; A=pΔ33-rSLO.3 pellet; A'=pΔ33-rSLO.3 supernatant; B=pBTac2-mSLO.3/6 pellet; B'=pBTac2-mSLO.3/6 supernatant; C=pBTac2-rSLO.3 pellet; C'=pBTac2-rSLO.3 supernatant.)

As is evident from Figure 9 itself, the pΔ33 expression system appears to differ from the pBTac2 system in that the bands for SLO-material, while appearing in approximately the same horizontal lane as with the pBTac2 system, appear to evidence a greater amount of SLO in the pellet compared to the pBTac2 system. In an effort to confirm these trends, the results of Figure 9 were analyzed by the aforemention Beckman DU 7000 spectrophotometer, and the resulting peaks for SLO-material (as a function of total peak area for both pellet and supernatant) for each system were compared. These results are summarized below in Table 4:

**TABLE 4**

| | pΔ33-rSLO.3 | | pBTac2-rSLO.3 | | pBTac2-mSLO.3/6 | |
|---|---|---|---|---|---|---|
| | S | P | S | P | S | P |
| | | | | | | |
| A | 9.23 | 8.14 | 13.71 | 7.01 | 12.44 | 7.22 |
| | | | | | | |
| B | 53 | 47 | 66 | 34 | 63 | 37 |

| | | | | | | |
|---|---|---|---|---|---|---|
| S - Supernantant | | | | | | |
| P - Pellet | | | | | | |
| A - Specific peak area relative to total peak areas for S lane and P lane | | | | | | |
| B - Percentage of (S + P) | | | | | | |

These results indicate that for the pΔ33 system, more SLO is in the pellet compared to the pBTac2 system; nearly twice as much SLO material is in the pBTac2 supernatant than in the pellet, while for the pΔ33 system, approximately the same amount of SLO material is in the supernatant and the pellet.

### Example 14

### Analysis of Specific Hemolytic Activity of Purified mSLO.3/6 and r/SLO.3

As noted, the specific activity and percent hemolytic activity of specific versions of rSLO and mSLO (purifed rSLO.3 and mSLO.3/6 respectively), based upon the "specific activity" of wild-type SLO, are as follows in Table 4:

**TABLE 5**

| | Wild-Type SLO | rSLO.3 | mSLO.3/6 |
|---|---|---|---|
| Specific Activity (Hemolytic Activity in Hemolytic Units/mg) | a) 1x10⁶ | | |
| | b) 1x10⁵ | 3.6x10⁴ | 14 |
| | | | |
| Percent Hemolytic Activity of Wild-Type SLO | a) 100 | 3.6 | 1.4x10⁻³ |
| | b) 100 | 9 | 3.5x10⁻³ |

The foregoing indicates that mSLO.3/6 is about 1.4x10⁻²% less hemolytically active than wild-type SLO. I.e., mSLO.3/6 has less than 1% of the hemolytic activity of wild-type SLO.

### Example 14

### In Vivo Toxicity Effects of mSLO.3/6

In order to evaluate in vivo toxicity effects of mSLO.3/6, Balb/c mice were administered undiluted and diluted intravenous injections of mSLO.3/6. Undiluted and diluted control suspension buffer was administered to an equivalent number of mice. To improve the intravenous injections, the mice were warmed under a heat lamp for 20-30 minutes of pre-injection. Approximately 20 mice were used for each condition.

For the undiluted mSLO.3/6, each mouse received an approximate dosage of 17 mg/kg, while for the diluted mSLO.3/6, each mouse received an approximate dosage of 1000 µg/kg. Control solution buffer did not affect the control mice.

Aside from minor ruffling for several minutes after injection, none of the mice receiving either diluted or undiluted mSLO.3/6 showed any ill effects from the intravenous administrations. This data supports the specific activity data for purified mSLO.3/6, i.e., the non-hemolytic activity of mSLO.3/6 is evident in that the mice receiving both diluted or undiluted doses thereof did not expire or otherwise evidence any serious side effects.

The foregoing Examples are directed to the generation of an SLO genomic library. As those in the art appreciate, another type of library which is much less complex than a genomic DNA library is a "complementary DNA", or "cDNA", library. cDNA is derived directly from mRNA; therefore, by definition, the cDNA library is comprised of regions of translation. Methods for deriving cDNA libraries based upon mRNA complementary to mSLO DNA are considered to be within the purview of the skilled artisan such that cDNA-based libraries for mSLO are a part of this disclosure.

### PROTEIN FOLDING

As the linear arrangement of nucleotides defines a specific codon, the arrangement, or sequence, of amino acids defines the protein, including the particular function thereof. However, while the particular amino acid sequence is important with respect to the identity of the protein, the particular three dimensional shape that the protein exhibits is of similar import. Such specificity in terms of shape, in essence, co-defines the properties of the protein because the shape of the protein enables the protein to specifically interact with other molecules that will only recognize that particular protein shape.

Most proteins spontaneously fold into their correct shape as they are translated within the cell. By treating the protein with certain denaturing solvents, the protein can "unfold" into a flexible chain. When the denaturing agent is removed, portions of the the flexible chain may refold into their original conformation. This is because one of the most important factors governing the folding of a protein is the distribution of polar (hydrophilic, or "water-hating") and non-polar (hydrophobic, or "water-loving") side chains of the amino acids of that protein. Denaturing solvents interfere with the polarity of the amino acid side chains. The following amino acids have polar side chains: Asn; Gln; Ser; Thr; and Tyr. The following amino acids have non-polar side chains: Gly; Ala; Val; Leu; Iso; Pro; Phe; Met; Trp; and Cys. Amino acids with basic and acidic side chains are very polar. The following amino acids have basis side chains: Lys; Arg; and His. The following amino acids have acidic side chains: Asp and Glu.

The environment in which proteins naturally exist is, by definition, a non-denaturing environment, which is most typically aqueous. Accordingly, the hydrophobic side chains of a protein tend to be pushed together in the interior of the protein molecule, which enables these to avoid contact with the aqueous environment. Polar side chains, on the other hand, tend to arrange themselves near the outside of the protein molecule, where they can interact with water and other polar molecules.

While the molecular mechanisms by which a linear DNA sequence is transcribed and translated into a precise amino acid sequence of the corresponding polypeptide is well understood, exactly how the polypeptide chain folds simultaneously and autonomously into its three-dimensional structure is not clearly understood. The real potential of synthetic DNA, i.e. DNA synthesized via recombinant techniques, will be realized in the area of of protein design. In order for this to be realized, however, the mechanism of protein folding will have to be more succinctly clarified. While the general problem of predicting protein structure from the sequence is elusive (principally because no rules have emerged that allow structure to be related to sequence), it is clear that certain portions of the sequence are important to the structure and other portions are relatively unimportant from a structural point of view such that substitutions or modifications can be made at these portions. Accordingly, it is assumed that portions of the sequence of a protein contribute significantly to the stability of the folded protein structure.

While predicting a protein structure from the protein sequence is elusive, proteins, by definition, have unique three-dimensional structures which can be determined. The following methodologies, for example, can be used in the determination of protein structure: Crystallography; Optical Activity, Nuclear Magnetic Resonance Spectroscopy.

### a) Crystollagraphy

Proteins are capable of forming crystals. Proteins usually crystallize in a condition of saturation or super-saturation which can be achieved by altering one or more of a number of variables that affect the solubility of the proteins. Thus, by altering the ionic strength of the solution or by utilization of organic polymers, e.g., polyethylene glycol, proteins can be crystallized. Techniques for growing protein crystals are set forth in Narang, S.A. Protein Engineering: Approaches to the Manipulation of Protein Folding (Butterworth, Publisher, Stoneham MA., 1990), Chpt. 6 (hereinafter "Narang"). The preceding text book is incorporated herein by reference in its entirety. Having crystallized the protein, the techniques of x-ray, neutron, and electron diffraction can be used to determine to structure of the protein, with x-ray diffraction being preferred. The protein structure in the crystal is assumed to be at or near the minimum conformational free energy of the molecule for the crystal form.

### b) Optical Activity

The optical activity of polypeptides/proteins due to the asymmetric centers of the amino acids and to the asymmetric conformations thereof, can be utilized to determine the structure of polypeptides/proteins. This asymmetry causes proteins to interact differently with right- and left-circularly polarized light; if the two beams consequently travel at different speeds through the protein, polarized light is rotated. Optical rotatory dispersion ("ORD") is the dependence of this rotation upon wavelength. In a wavelength region where the protein molecule does not absorb light, the rotation varies gradually with wavelength, but in an absorbance region, the rotation first increases sharply in one direction, falls to zero at the absorption maximum, and then rises sharply in the opposite direction. There will also be unequal absorption of left- and right-circularly polarized light; this is referred to as circular dichroism ("CD"). Both CD and OES spectra of a protein are very sensitive to the structural conformation thereof. Folded proteins generally have significant optical activity in the near-UV region (250-300nm).

### c) Nuclear Magnetic Resonance Spectroscopy

Nuclear Magnetic Resonance Spectroscopy, using, e.g., ¹H, ¹³C, ¹⁵N, ¹³P or ²H, has proven to be of great use in studying protein structure in solution. Focusing on 1H, each hydrogen atom in a molecule has a nuclear magnetic spin, i.e. the nuclei of the atom act like tiny magnets. In the absence of an external magnetic field, the magnetic moments of the protons are randomly oriented. In a Nuclear Magnetic Resonance experiment, a strong external magnetic field is applied to the sample along a specified direction, resulting in a net alignment of the magnetic moments and a net macroscopic magnetization along the specified directional axis; a short radio-frequency pulse of appropriate strength is then applied, knocking the magnetization vector away from this axis. As the magnetization recovers, a transient radio-frequency signal is recorded as a function of time. A fourier-transform of this signal then yields a frequency spectrum. Each proton in the molecule gives rise to a peak in this spectrum occurring at some characteristic resonance frequency determined by the local electronic environment of that proton. The resonance frequency of a particular proton is called its "chemical shift" and is measured as an offset from some reference frequency. Structured information from NMR is derived from the nuclear Overhauser effect ("NOE", which determines whether a pair of protons are near each other in space) and the coupling constants of protons that are separated by three or fewer chemical bonds. NOE and coupling constants provide one-dimensional data; two-dimensional data is provided by inter alia nuclear Overhauser enhancement spectroscopy (NOESY) and two-dimensional correlation spectroscopy (COSY); and from such data, three-dimensional protein structures can be determined.

In view of the foregoing information set forth with respect to determination of the three-dimensional structure of protein molecules, the following claims directed to DNA macromolecules and amino acids inherently include the three dimensional structures associated with the protein molecules expressed thereby.

The Examples herein are not to be construed as limited to specific vectors, plasmids and host cells which are preferred. The mSLO described herein is not to be construed as limited solely to the preferred mSLO designated mSLO.3/6. Similarly, the preferred mSLO.3/6 in no way constitute an admission, either actual or implied, that the DNA and amino acid sequences thereof are the only DNA and amino acid sequences to which Applicant is entitled. Applicant is entitled to the full breadth of protection under applicable patent laws.

For purposes of claiming materials by designation, JM105 transformed with plasmids comprising pBTac2 DNA - mSLO.3/6 and AR120 transformed with vectors comprising pΔ33 - mSLO.3/6 were deposited on August 23, 1991 with the American Type Culture Collection (ATCC), 12301 Parklawn Drive, Rockville, Maryland, 20852, under the provisions of the Budapest Treaty for the International Recognition of the Deposit of Microorganisms for the Purpose of Patent Procedure. These were tested by the ATCC on August 27, 1991, and determined to both be viable. The ATCC has assigned the deposit numbers ATCC 68678 and ATCC 68676, respectively, to these materials.

Having identified a single nucleotide (and aminoacid) difference between rSLO.3 and mSLO.3/6, those skilled in the art can readily prepare the DNA sequence set forth in Figure 1 using various methodologies known to those in the art, e.g., wild-type SLO subjected to the process described in, e.g., United States Patent No. 4,351,901 ("Method for single nucleotide alteration"), which is incorporated herein by reference. Other methodologies for nucleotide and amino acid substitution, terminal and intermediate alterations and deletions will be readily apparent to those skilled in the art. Furthermore, as the DNA synthesis art progresses such that oligonucleotides having the length of the DNA sequence of Figure 1 can be rapidly obtained, one can synthesize that sequence as appropriate with such advances in the art.

Additionally, because Applicant has discovered. inter alia, that the single nucleotide substitution identified in Figure 1 (which in turn leads to a single amino acid change as set forth in Figure 2) has reduced the hemolytic activity of the SLO variant as described, those skilled in the art can readily obtain fragments of that DNA sequence (e.g., via nucleotide deletion) such that the fragment continues to maintain at least one epitopic site characteristic of wild-type SLO and maintains non-hemolytic characteristics. Furthermore, and as noted, conservative substitutions of nucleotides can be made without concomitant changes in the amino acid sequence, as those in the art understand and appreciate. For example,"computerized back translation" techniques can be used, whereby the amino acid sequence is analyzed by a computer and the computer determines the optimum nucleotides to utilize in the codons necessary to encode such amino acids. Additionally, DNA sequences preferably evidencing 80% homology with the DNA sequence of Figure 1, preferably 85%, and most preferably 90% homology with that sequence are considered to fall within the scope of the invention.

Because the screening of SLO variants can be readily accomplished using the above-described blood overlay technique, numerous SLO variant candidates can be rapidly evaluated. Accordingly, having defined the specific substitution that has led to the specific SLO variant, those skilled in the art can readily use this advance in the art to derive SLO variant analog candidates, rapidly screen these candidates for indications of non-hemolytic activity, and determine the nucleic acid and amino acid sequences of analogs which fall within Applicant's definition of mSLO.

Accordingly: while the Examples herein are directed to a specific SLO variant, mSLO.3/6; because, statistically speaking, this variant has no hemolytic activity compared to wild-type SLO; and because having had this advance in the art placed in their possession, those in the art can utilize techniques known to the art to adapt this advance to their own ends, Applicant's invention is seen to comprehend SLO variants having the characteristics as defined, and is not limited to the specific variant disclosed in the Examples.

Although the present invention has been described in considerable detail with regard to certain preferred embodiments thereof, other embodiments within the scope of the teachings of the present invention are possible. As such, while the production of a specific SLO variant has been described in detail, this is to be construed as an exemplar. Accordingly, neither the disclosure, nor the claims to follow, are intended, nor should be construed to be, limited by the descriptions of the preferred embodiments contained herein.

## Claims

1. A purified and isolated DNA sequence encoding a variant of Streptolysin O, said DNA sequence having at least one single base change from wild-type Streptolysin O such that the encoded variant of Streptolysin O is soluble and substantially non-hemolytic.

2. The DNA sequence of claim 1 wherein the variant of Streptolysin O is characterized by evidencing less than about 1.5% of the hemolytic activity of wild-type Streptolysin O, said wild-type Streptolysin O having a hemolytic activity of about 4 x 10⁵ hemolytic units per milligram.

3. The DNA sequence of claim 1 wherein the variant of Streptolysin O is characterized by evidencing less than about 0.5% of the hemolytic activity of wild-type Streptolysin O, said wild-type Streptolysin O having a hemolytic activity of about 4 x 10⁵ hemolytic units per milligram.

4. The purified and isolated DNA sequence of claim 1 wherein the variant of Streptolysin O is characterized by evidencing less than about 0.1% of the hemolytic activity of wild-type Streptolysin O, said wild-type Streptolysin O having a hemolytic activity of about 4 x 10⁵ hemolytic units per milligram.

5. A DNA sequence encoding a soluble, substantially non-hemolytic variant version of Streptolysin O comprising the DNA sequence as set forth in Figure 1 from nucleotide 7 through nucleotide 1524, inclusive.

6. A prokaryotic or eukaryotic host cell transformed or transfected with a DNA sequence according to claim 1, 2, 3, 4 or 5 in a manner allowing the host cell to express a variant version of Streptolysin O.

7. A DNA plasmid consisting of a DNA vector and a DNA sequence according to claim 1, 2, 3, 4 or 5.

8. The DNA plasmid of claim 7 wherein said DNA vector further comprises a promoter.

9. The DNA plasmid of claim 7 wherein said DNA vector further comprises a regulated promoter.

10. The DNA plasmid of claim 7 wherein said DNA vector further comprises a regulated promoter selected from P_{L}, P_{tac}, and P_{T7}.

11. A prokaryotic or eukaryotic host cell transformed or transfected with a DNA plasmid according to any one of claims 7 to 10.

12. An amino acid sequence encoding a soluble, substantially non-hemolytic variant of Streptolysin O consisting of amino acids 1 through 473, inclusive, of the amino acid sequence set forth in Figure 2.

13. A cDNA sequence according to claim 1.

14. A prokaryotic or eukaryotic host cell transformed or transfected with a DNA sequence according to claim 13 in a manner allowing the host cell to express a variant of Streptolysin O.

15. A DNA plasmid comprising a DNA vector and a DNA sequence according to claim 13.

16. A prokaryotic or eukaryotic host cell transformed or transfected with a DNA plasmid according to claim 15.

17. The DNA plasmid of claim 15 wherein said DNA vector further comprises a promoter.

18. The DNA plasmid of claim 15 wherein said DNA vector further comprises a regulated promoter.

19. The DNA plasmid of claim 15 wherein said DNA vector further comprises a regulated promoter selected from P_{L}, P_{tac} and P_{T7}.

20. A purified soluble, substantially non-hemolytic variant version of Streptolysin O comprising at least one epitope characteristic of wild-type Streptolysin O, said soluble variant contains at least one single amino acid change such that the variant is capable of being soluble upon expression and evidencing less than about 1.5% of the hemolytic activity of wild-type Streptolysin O; and said variant having a hemolytic activity of about 4 x 10⁵ hemolytic units per milligram of said wild-type Streptolysin O.

21. The variant version of Streptolysin O of claim 20 evidencing less than about 0.5% of the hemolytic activity of wild-type Streptolysin O, said wild-type Streptolysin O having a hemolytic activity of about 4 x 10⁵ hemolytic units per milligram.

22. The variant version of Streptolysin O of claim 20 evidencing less than about 0.1% of the hemolytic activity of wild-type Streptolysin O, said wild-type SLO having a hemolytic activity of about 4 x 10⁵ hemolytic units per milligram.

23. A purified soluble, substantially non-hemolytic variant version of Streptolysin O having a specific hemolytic activity of less than about 400 HU/mg and being soluble upon expression, said soluble variant contains at least one single amino acid change such that the variant is substantially non-hemolytic.

24. A process for obtaining a purified soluble, substantially non-hemolytically active variant of Streptolysin O which is soluble upon expression comprising the steps of:
(a) introducing a fragment consisting of a purified isolated DNA sequence encoding a derivative of Streptolysin O into a host, said DNA sequence having at least one deletion from wild-type Streptolysin O such that the encoded derivative of Streptolysin O is soluble and hemolytic, said host capable of producing high levels of mutagenic activity, to obtain a mutation fragment candidate having at least one single base change from wild-type Streptolysin O such that the encoded variant of Streptolysin O is soluble and substantially non-hemolytic;
(b) introducing said mutation fragment candidate into an appropriate vector to obtain a plasmid;
(c) transforming a host capable of expressing the mutation fragment candidate with the plasmid to obtain a mutation protein candidate; and
(d) screening said mutation protein candidate for an absence of hemolysis of red blood cells.

25. The DNA sequence of claims 1 or 13, wherein the single base change occurs at codon 487 of the DNA sequence as set forth in Figure 1 from nucleotide 7 through nucleotide 1524, inclusive.

26. The variant of claim 20 or 23, wherein the single amino acid change is from glutamic acid to lysine at amino acid 161 of amino acids 1 through 473, inclusive, of the amino acid sequence set forth in Figure 2.

## Patentansprüche

1. Gereinigte und isolierte DNA-Sequenz, welche eine Variante von Streptolysin O kodiert, wobei diese DNA-Sequenz wenigstens einen einzigen Basenaustausch des Wild-Typs des Streptolysin O in der Art hat, daß die kodierte Variante von Streptolysin O löslich und im wesentlichen nicht-hemolytisch ist.

2. DNA-Sequenz nach Anspruch 1, bei der die Variante von Streptolysin O durch nachweisbar weniger als ungefähr 1,5 % der hemolytischen Aktivität des Wild-Typs von Streptolysin O charakterisiert ist, wobei dieser Wild-Typ von Streptolysin O eine hemolytische Aktivität von ungefähr 4 x 10⁵ hemolytische Einheiten pro Milligramm hat.

3. DNA-Sequenz nach Anspruch 1, bei der die Variante des Streptolysin O durch nachweisbar weniger als ungefähr 0,5 % der hemolytischen Aktivität des Wild-Typs von Streptolysin O charakterisiert ist, wobei der Wild-Typ von Streptolysin O eine hemolytische Aktivität von ungefähr 4 x 10⁵ hemolytischen Einheiten pro Milligramm hat.

4. Gereinigte und isolierte DNA-Sequenz nach Anspruch 1, bei der die Variante von Streptolysin O durch nachweisbar weniger als ungefähr 0,1 % der hemolytischen Aktivität des Wild-Typs von Streptolysin O charakterisiert ist, wobei der Wild-Typ von Streptolysin O eine hemolytische Aktivität von ungefähr 4 x 10⁵ hemolytischen Einheiten pro Milligramm hat.

5. DNA-Sequenz, die eine lösliche, im wesentlichen nicht-hemolytische Variantenversion von Streptolysin O kodiert, welche die DNA-Sequenz, gemäß Fig. 1 von Nukleotid 7 bis einschließlich Nukleotid 1524 beinhaltet.

6. Prokaryote oder eukaryote Wirts-Zelle, die durch eine DNA-Sequenz nach Anspruch 1, 2, 3, 4 oder 5 in der Weise transformiert oder transfiziert wurde, welche der Wirts-Zelle die Expression einer Variantenversion von Streptolysin O gestattet.

7. DNA-Plasmid, der aus einem DNA-Vektor und einer DNA-Sequenz gemäß Anspruch 1, 2, 3, 4 oder 5 besteht.

8. DNA-Plasmid nach Anspruch 7, bei dem dieser DNA-Vektor des weiteren einen Promoter beinhaltet.

9. DNA-Plasmid nach Anspruch 7, bei dem dieser DNA-Vektor des weiteren einen geregelten Promoter beinhaltet.

10. DNA-Plasmid nach Anspruch 7, bei dem dieser DNA-Vektor des weiteren einen geregelten Promoter ausgewählt unter P_{L}, P_{tac}, und P_{T7} beinhaltet.

11. Prokaryote oder eukaryote Wirts-Zelle, die durch ein DNA-Plasmid nach einem der Ansprüche 7 bis 10 transformiert oder transfiziert wurde.

12. Aminosäure-Sequenz, die eine lösliche, im wesentlichen nicht-hemolytische Variante von Streptolysin O kodiert, welche aus Aminosäuren 1 bis einschließlich 473 der Aminosäure-Sequenz nach Fig. 2 besteht.

13. Eine cDNA-Sequenz nach Anspruch 1.

14. Prokaryote oder eukaryote Wirts-Zelle, die mit einer DNA-Sequenz nach Anspruch 13 in einer Weise transformiert oder transfiziert wurde, welche der Wirts-Zelle gestattet, eine Variante des Streptolysin O zu exprimieren.

15. DNA-Plasmid, mit einem DNA-Vektor und einer DNA-Sequenz nach Anspruch 13.

16. Prokaryote oder eukaryote Wirts-Zelle, die durch ein DNA-Plasmid nach Anspruch 15 transformiert oder transfiziert wurde.

17. DNA-Plasmid nach Anspruch 15, bei dem dieser DNA-Vektor des weiteren einen Promoter beinhaltet.

18. DNA-Plasmid nach Anspruch 15, bei dem dieser DNA-Vektor des weiteren einen geregelten Promoter beinhaltet.

19. DNA-Plasmid nach Anspruch 15, bei dem dieser DNA-Vektor des weiteren einen geregelten Promoter ausgewählt unter P_{L}, P_{tac}, und P_{T7} beinhaltet.

20. Gereinigte, lösliche, im wesentlichen nicht-hemolytische Variantenversion von Streptolysin O, die wenigstens ein Epitop charakteristisch für den Wild-Typ des Streptolysin 0 beinhaltet, wobei diese lösliche Variante wenigstens einen einzelnen Austausch einer Aminosäure der Art enthält, daß die Variante bei der Expression löslich sein kann und weniger als ungefähr 1,5 % der hemolytischen Aktivität des Wild-Types von Streptolysin O zeigt; und wobei diese Variante eine hemolytische Aktivität von ungefähr 4 x 10⁵ hemolytische Einheiten pro Milligramm von diesem Wild-Typ des Streptolysin O hat.

21. Variantenversion von Streptolysin O nach Anspruch 20, die nachweislich weniger als ungefähr 0,5 % der hemolytischen Aktivität des Wild-Typs von Streptolysin O hat, wobei der Wild-Typ von Streptolysin O eine hemolytische Aktivität von ungefähr 4 x 10⁵ hemolytischen Einheiten pro Milligramm hat.

22. Variantenversion von Streptolysin O nach Anspruch 20, die nachweislich weniger als ungefähr 0,1 % der hemolytischen Aktivität des Wild-Typs von Streptolysin O hat, wobei dieser Wild-Typ von SLO eine hemolytische Aktivität von ungefähr 4 x 10⁵ hemolytischen Einheiten pro Milligramm hat.

23. Gereinigte, lösliche, im wesentlichen nicht-hemolytische Variantenversion von Streptolysin O, die eine spezifische hemolytische Aktivität von weniger als ungefähr 400 HU/mg hat und bei der Expression löslich ist, wobei die lösliche Variante wenigstens einen Austausch einer einzigen Aminosäure der Art hat, daß die Variante im wesentlichen nicht-hemolytisch ist.

24. Verfahren zur Gewinnung einer gereinigten, löslichen, im wesentlichen nicht-hemolytisch aktiven Variante von Streptolysin O, welche bei der Expression löslich ist, mit den folgenden Schritten:
(a) Einführung eines Fragmentes bestehend aus einer gereinigten, isolierten DNA-Sequenz, welche ein Derivat des Streptolysin O in einem Wirt kodiert, wobei diese DNA-Sequenz wenigstens eine Deletion vom Wild-Typ des Streptolysin O in der Art hat, daß das kodierte Derivat von Streptolysin O löslich und hemolytisch ist, wobei dieser Wirt hohe Spiegel an mutagener Aktivität produzieren kann, um einen Kandidaten für ein Mutations-Fragment zu erhalten, der wenigstens einen einzigen Basenaustausch vom Wild-Typ des Streptolysin O in der Art hat, daß die kodierte Variante von Streptolysin O löslich und im wesentlichen nicht-hemolytisch ist;
(b) Einführen dieses Kandidatens eines Mutations-Fragmentes in einen geeigneten Vektor, um ein Plasmid zu gewinnen;
(c) Transformation eines Wirts, der den Kandidaten des Mutations-Fragmentes exprimieren kann, mit dem Plasmid, um einen Kandidaten für ein mutiertes Protein zu erhalten; und
(d) Untersuchung dieses Kandidaten für ein mutiertes Protein auf Abwesenheit von Hemolyse der roten Blutzellen.

25. DNA-Sequenz nach Anspruch 1 oder 13, worin der einzige Basenwechsel am Codon 487 der DNA-Sequenz wie in Fig. 1 von Nukleotid 7 bis einschließlich Nukleotid 1524 erfolgt.

26. Variante nach Anspruch 20 oder 23, bei der der einzige Aminosäureaustausch von Glutaminsäure nach Lysin bei Aminosäure 161 der Aminosäuren 1 bis einschließlich 473 der Aminosäure-Sequenz nach Fig. 2 erfolgt.

## Revendications

1. Séquence d'ADN purifiée et isolée codant un variant de Streptolysine O, ladite séquence d'ADN ayant au moins un seul changement de base par rapport à la Streptolysine O du type sauvage tel que le variant codé de la Streptolysine O soit soluble et sensiblement non-hémolytique.

2. Séquence d'ADN de la revendication 1 où le variant de Streptolysine O est caractérisé par la mise en évidence de moins d'environ 1,5% de l'activité hémolytique de la Streptolysine O du type sauvage, ladite Streptolysine O du type sauvage ayant une activité hémolytique d'environ 4 x 10⁵ unités hémolytiques par milligramme.

3. Séquence d'ADN de la revendication 1 où le variant de la Streptolysine O est caractérisé par la mise en évidence de moins d'environ 0,5% de l'activité hémolytique de la Streptolysine O du type sauvage, ladite Streptolysine O du type sauvage ayant une activité hémolytique d'environ 4 x 10⁵ unités hémolytiques par milligramme.

4. Séquence d'ADN purifiée et isolée de la revendication 1 où le variant de la Streptolysine O est caractérisé par la mise en évidence de moins d'environ 0,1% de l'activité hémolytique de la Streptolysine O du type sauvage, ladite Streptolysine O du type sauvage ayant une activité hémolytique d'environ 4 x 10⁵ unités hémolytiques par milligramme.

5. Séquence d'ADN codant pour une version d'un variant soluble sensiblement non hémolytique de la Streptolysine O comprenant la séquence d'ADN telle que montrée à la Figure 1 du nucléotide 7 au nucléotide 1524, inclus.

6. Cellule hôte procaryote ou eucaryote transformée ou transfectée avec une séquence d'ADN selon la revendication 1, 2, 3, 4 ou 5 d'une manière permettant à la cellule hôte d'exprimer une version d'un variant de Streptolysine O.

7. Plasmide d'ADN consistant en un vecteur d'ADN et une séquence d'ADN selon la revendication 1, 2, 3, 4 ou 5.

8. Plasmide d'ADN de la revendication 7 où ledit vecteur d'ADN comprend de plus un promoteur.

9. Plasmide d'ADN de la revendication 7 où ledit vecteur d'ADN comprend de plus un promoteur régulé.

10. Plasmide d'ADN de la revendication 7 où ledit vecteur d'ADN comprend de plus un promoteur régulé sélectionné parmi P_{L}, P_{tac}, et P_{T7}.

11. Cellule hôte procaryote ou eucaryote transformée ou transfectée avec un plasmide d'ADN selon l'une quelconque des revendications 7 à 10.

12. Séquence d'acides aminés codant pour un variant soluble et sensiblement non hémolytique de la Streptolysine O consistant en acides aminés 1 à 473, inclus, de la séquence d'acides aminés montrée à la Figure 2.

13. Séquence d'ADNc selon la revendication 1.

14. Cellule hôte procaryote ou eucaryote transformée ou transfectée avec une séquence d'ADN selon la revendication 13 d'une manière permettant à la cellule hôte d'exprimer un variant de la Streptolysine O.

15. Plasmide d'ADN comprenant un vecteur d'ADN et une séquence d'ADN selon la revendication 13.

16. Cellule hôte procaryote ou eucaryote transformée ou transfectée avec un plasmide d'ADN selon la revendication 15.

17. Plasmide d'ADN de la revendication 15 où ledit vecteur d'ADN comprend de plus un promoteur.

18. Plasmide d'ADN de la revendication 15 où ledit vecteur d'ADN comprend de plus un promoteur régulé.

19. Plasmide d'ADN de la revendication 15 où ledit vecteur d'ADN comprend de plus un promoteur régulé sélectionné parmi P_{L}, P_{tac}, et P_{T7}.

20. Version d'un variant purifié soluble, sensiblement non-hémolytique de la Streptolysine O comprenant au moins un épitope caractéristique de la Streptolysine O du type sauvage, ledit variant soluble contient au moins un seul changement d'acide aminé tel que le variant soit capable d'être soluble lors d'une expression et mettant en évidence moins d'environ 1,5% de l'activité hémolytique de la Streptolysine O du type sauvage; et ledit variant ayant une activité hémolytique d'environ 4 x 10⁵ unités hémolytiques par milligramme de ladite Streptolysine O du type sauvage.

21. Version du variant de la Streptolysine O de la revendication 20 mettant en évidence environ 0,5% de l'activité hémolytique de la Streptolysine O du type sauvage, ladite Streptolysine O du type sauvage ayant une activité hémolytique d'environ 4 x 10⁵ unités hémolytiques par milligramme.

22. Version du variant de la Streptolysine O de la revendication 20 mettant en évidence moins d'environ 0,1% de l'activité hémolytique de la Streptolysine O du type sauvage, ladite SLO du type sauvage ayant une activité hémolytique d'environ 4 x 10⁵ unités hémolytiques par milligramme.

23. Version d'un variant purifié soluble sensiblement non-hémolytique de la Streptolysine O ayant une activité hémolytique spécifique de moins d'environ 400 UH/mg et étant soluble lors d'une expression, ledit variant soluble contient au moins un seul changement d'acide aminé tel que le variant soit sensiblement non-hémolytique.

24. Procédé d'obtention d'un variant purifié soluble sensiblement non-hémolytiquement actif de la Streptolysine O qui est soluble lors d'une expression, comprenant les étapes de:
(a) introduire un fragment consistant en une séquence d'ADN purifiée isolée codant pour un dérivé de la Streptolysine O dans un hôte, ladite séquence d'ADN ayant au moins une délétion par rapport à la Streptolysine O du type sauvage telle que le dérivé codé de la Streptolysine O soit soluble et hémolytique, ledit hôte étant capable de produire des niveaux élevés d'activité de mutagénèse, pour obtenir un fragment candidat à la mutation ayant au moins un seul changement de base par rapport à la Streptolysine O du type sauvage de manière que le variant codé de la Streptolysine O soit soluble ou sensiblement non-hémolytique;
(b) introduire ledit fragment candidat de mutation dans un vecteur approprié pour obtenir un plasmide;
(c) transformer un hôte capable d'exprimer le fragment candidat de mutation avec le plasmide pour obtenir une protéine candidate de mutation; et
(d) cribler ladite protéine candidate de mutation à la recherche de l'absence d'hémolyse des globules rouges.

25. Séquence d'ADN des revendications 1 ou 13, où le seul changement d'une base se produit au codon 487 de la séquence d'ADN telle que montrée à la Figure 1 à partir du nucléotide 7 jusqu'au nucléotide 1524, inclus.

26. Variant de la revendication 20 ou 23, où le seul changement d'acide aminé est de l'acide glutamique à la lysine à l'acide aminé 161 des acides aminés 1 à 473, inclus, de la séquence d'acides aminés montrée à la Figure 2.
